# FASCICULE DE BREVET EUROPEEN

(11) **EP 3 574 120 B1**
(45) Date de publication et mention de la délivrance du brevet: **06.04.2022**
(21) Numéro de dépôt: 18712080.3
(22) Date de dépôt: 24.01.2018
(51) Int. Cl.: C12N 1/22, C12N 15/01, C12N 15/52, C12P 7/06, C12N 1/18, C12P 1/02, C12P 7/10, C12R 1/865

(54) **OBTENTION DE SOUCHES DE LEVURE PERFORMANTES POUR LA METABOLISATION DE L'ARABINOSE**
ERZEUGUNG VON HEFE-STRAINS ZUR METABOLISIERUNG DER ARABINOSE
OBTAINING PERFORMING YEAST STRAINS FOR METABOLIZATION OF ARABINOSIS

(30) Priorité: 24.01.2017 FR 1750550
(43) Date de publication de la demande: 04.12.2019
(73) Titulaire: Lesaffre et Compagnie, 75001 Paris (FR)
(72) Inventeur: DESFOUGERES, Thomas, 86130 Dissay (FR); FRITSCH, Emilie, NW5 3BX London (GB); PIGNEDE, Georges, 59700 Marcq en Baroeul (FR); RAVE, Christophe, 59130 Lambersart (FR); THOREL, Claire, 59223 Roncq (FR)
(74) Mandataire: Cabinet Laurent & Charras
(86) Numéro de dépôt international: PCT/EP2018/051708
(87) Numéro de publication internationale: WO 2018/138135

(56) Documents cités:
- WO-A1-2008/041840
- WO-A1-2013/178915
- WO-A1-2014/207087
- WO-A2-2009/112472
- WO-A2-2012/143513
- FR-A1- 3 035 405
- US-A1- 2014 206 070
- WANG CHENGQIANG ET AL: "Improvement of L-arabinose fermentation by modifying the metabolic pathway and transport in Saccharomyces cerevisiae.", BIOMED RESEARCH INTERNATIONAL, vol. 2013, 461204, 2013, pages 1-9, XP002774755, ISSN: 2314-6141, DOI: 10.1155/2013/461204
- MADHAVAN ANJALI ET AL: "Bioconversion of lignocellulose-derived sugars to ethanol by engineered Saccharomyces cerevisiae", CRITICAL REVIEWS IN BIOTECHNOLOGY, vol. 32, no. 1, mars 2012 (2012-03), pages 22-48, XP002774751,
- WISSELINK H WOUTER ET AL: "Novel Evolutionary Engineering Approach for Accelerated Utilization of Glucose, Xylose, and Arabinose Mixtures by Engineered Saccharomyces cerevisiae Strains", APPLIED AND ENVIRONMENTAL MICROBIOLOGY, AMERICAN SOCIETY FOR MICROBIOLOGY, US, vol. 75, no. 4, 1 février 2009 (2009-02-01), pages 907-914, XP009121294, ISSN: 0099-2240, DOI: 10.1128/AEM.02268-08

## Description

### DOMAINE DE L'INVENTION

La présente demande a trait à des souches de levure aptes à métaboliser l'arabinose, en association avec leur aptitude à fermenter le xylose et le glucose, y compris en présence d'inhibiteurs de ces deux dernières fermentations, à savoir l'acide acétique sous forme non dissociée.

Plus précisément, la présente demande offre un procédé permettant de sélectionner des souches aptes à métaboliser l'arabinose et des souches améliorées, performantes dans leur capacité à métaboliser l'arabinose mais aussi le xylose, ces deux types de pentoses étant retrouvés dans les hydrolysats lignocellulosiques.

### ETAT DE LA TECHNIQUE

La biomasse lignocellulosique ou végétale, issue essentiellement de l'activité agricole et agro-industrielle, est un substrat complexe, constitué de trois principales fractions que sont la cellulose, les hémicelluloses et la lignine. Il s'agit d'un déchet recyclable, utile pour la production d'éthanol dont la demande, en vue par exemple de son utilisation en tant que biocarburant, ne cesse de croitre.

Le procédé de production d'éthanol à partir de la biomasse lignocellulosique consiste à récupérer par hydrolyse le maximum de sucres présents dans les fractions cellulosiques et hémicellulosiques puis de les transformer en éthanol par fermentation.

Concernant la fermentation des sucres présents dans cette biomasse comprenant à la fois des sucres en C6 (hexoses) ou des sucres en C5 (pentoses), il est aujourd'hui favorisé la fermentation anaérobie par les levures, en particulier à l'aide de *Saccharomyces cerevisiae* dont la capacité à fermenter le glucose en éthanol est largement maîtrisée et exploitée.

Toutefois, toute l'attention s'est portée sur la fermentation des pentoses, notamment le xylose, qui peuvent représenter jusqu'à 25 à 40% des sucres totaux contenus dans la biomasse lignocellulosique. Ainsi, des souches de levure aptes à fermenter le glucose ont été modifiées pour pouvoir également métaboliser les pentoses.

A titre d'exemple, le document WO 2010/000464 rapporte l'obtention de souches de levure aptes à fermenter les pentoses grâce à un gène bactérien codant une xylose isomérase (XI) qui convertit le xylose en xylulose métabolisable par la levure.

A noter que de manière alternative, une voie comprenant une xylose réductase (XR ou *XYL1*) générant du xylitol et une xylitol déshydrogénase (XDH ou *XYL2)* permet également d'aboutir au xylulose.

Ainsi, le document WO 2012/072793 décrit des souches de levure améliorées, associant des gènes exogènes codant une xylose isomérase et une xylitol déshydrogénase permettant d'éliminer le xylitol qui s'avère être un inhibiteur de la xylose isomérase. De telles souches, en particulier la souche déposée à la CNCM le 5.10.2011 sous le numéro I-4538, présentent des rendements améliorés et donc une utilité industrielle avérée pour la production d'éthanol.

Une autre problématique cruciale a été la mise en évidence, dans les hydrolysats lignocellulosiques, d'inhibiteurs de la fermentation parmi lesquels figurent des furaldéhydes (furfural, HMF), des composés phénoliques et des acides organiques (acide acétique, acide lévulinique, acide formique, ...). En particulier, la présence de forte concentration en acide acétique, supérieure à 5 g/kg (de milieu initial) et pouvant atteindre jusqu'à 10 g/kg, est intrinsèquement liée à celle de groupements acétyles associés de manière covalente avec les molécules d'hémicellulose.

Des travaux antérieurs ont abordé l'amélioration de la résistance des souches vis-à-vis de la présence d'acide acétique dans les mouts de fermentation. Ainsi, le document WO 2011/080411 a rapporté l'obtention de souches de levure dont la résistance à l'acide acétique sur glucose a été améliorée.

Cependant, l'acide acétique est également un inhibiteur de la fermentation du xylose. Cette inhibition se caractérise par une réduction de la cinétique de consommation du xylose (Bellisimi et al., FEMS Yeast Res., 2009, 9 :358-364), alors que sur le glucose, cette inhibition se traduit par un retard lors de l'initiation de la fermentation, la cinétique restant par la suite inchangée. A noter qu'en présence à la fois de glucose et de xylose dans le milieu, les souches de levure fermentent en priorité le glucose en raison de la répression catabolique.

Ainsi, les documents WO 2013/178915 et WO 2013/178918 décrivent des procédés d'obtention de souches de levure aptes à métaboliser les pentoses, notamment le xylose, et résistantes aux inhibiteurs de fermentation, en particulier l'acide acétique.

Des souches encore améliorées, en particulier dans leur capacité à fermenter le glucose et le xylose en présence d'acide acétique, sont décrites dans les documents WO 2015/121595 et FR 3 035 405.

L'ensemble de ces travaux se sont concentrés sur le xylose comme pentose d'intérêt. Même s'il est généralement considéré comme le pentose majoritaire dans l'hémicellulose, le xylose n'y est pas le seul. Ainsi, il est possible de trouver également de l'arabinose dans ces structures végétales (Saddler, 1993, Wallingford, Oxon, UK: CAB International), dans une proportion parfois supérieure à 40 % (Schädel et al., 2010, Physiologia Plantarum 139, 241-255). Par ailleurs, l'arabinose se retrouve dans la bagasse (Hanko and Rohrer, 2000, Anal. Biochem. 283, 192-199) ou dans des fibres issues du maïs appelées « corn fibers » (Gulati et al., 1996, Bioresource Technology 58, 253-264).

Outre l'intérêt économique évident de la fermentation supplémentaire de l'arabinose, il est également à noter que l'arabinose résiduel peut constituer un substrat de choix pour le développement de microorganismes contaminants, éventuellement capables de le convertir en acides organiques qui, comme déjà dit, sont des inhibiteurs de la fermentation du glucose et du xylose (Schell et al., 2007, Bioresour. Technol. 98, 2942-2948).

De nombreux microorganismes ont été décrits comme étant capables de métaboliser l'arabinose aussi bien chez les microorganismes fongiques, en particulier les hémi-ascomycètes tels que *Scheffersomyces stipitis,* les basidiomycètes et les champignons filamenteux, que chez les bactéries telles que *Erwinia chrysanthemi, Thermoanaerobacterium saccharolyticum, Escherichia coli, Zymomonas mobilis, Bacillus subtilis, Bacillus licheniformis* et *Lactobacillus plantarum.*

Toutefois, parmi les levures exploitables au niveau industriel, notamment celles capables de supporter des titres en éthanol aussi élevés que ceux tolérés par la levure *Saccharomyces cerevisiae,* aucune n'a été rapportée comme étant naturellement capable de fermenter l'arabinose.

Le document WO 03/095627 a rapporté la possibilité d'obtenir des souches de *S. cerevisiae* capables de croitre sur arabinose en transformant une souche de laboratoire par des plasmides multicopies portant spécifiquement les gènes *araA* (codant une L-arabinose isomérase) de *B. subtilis, araB* (codant une L-ribulokinase avantageusement mutée) et *araD* (codant une L-ribulose-5-P-4 épimérase) d'E. *coli.* Ce document pose ouvertement la question de la possibilité d'obtenir une souche capable de co-fermenter le xylose.

Le document WO 2008/122354 décrit des souches de *S. cerevisiae* transformées à l'aide de vecteurs auto-réplicatifs portant les gènes *araA* de *B. licheniformis, araB* (avantageusement muté) et *araD d'E. coli,* avantageusement codon-optimisés, capables de croitre en aérobiose ou en anaérobiose dans des milieux contenant comme seule source de carbone de l'arabinose. La sélection des transformants est réalisée par culture dans un milieu contenant du glucose comme source de carbone.

Le document Wang et al. (2013, Biomed Research International, 2013, 1-9) décrit la sélection de souches de *S. cerevisiae,* intégrant au niveau chromosomique les gènes *araA, araB* et *araD* de *L. plantarum* codon-optimisés et surexprimant *GAL2,* pour leur capacité à métaboliser l'arabinose.

Le document WO 2008/041840 décrit des séquences spécifiques de *araA, araB* et *araD* conférant la capacité à une levure de métaboliser l'arabinose, en l'occurrence issues de *L. plantarum.* En pratique, ces gènes sont introduits via des plasmides dans une souche de laboratoire de *S. cerevisiae* délétée pour le gène de l'aldose réductase *GRE3,* surexprimant la voie des pentoses phosphates (*TAL1, TKL1, RPE1* et *RKI1*) et exprimant la voie de fermentation du xylose (*XI ou XylA* et *XKS1*). D'après les auteurs, une telle souche n'est pas capable de pousser directement sur un milieu contenant de l'arabinose et un prépassage sur un milieu contenant du galactose est requis. En outre, les souches obtenues à l'issue de ces cultures ont perdu leur capacité à métaboliser le xylose.

Le document WO 2012/143513 rapporte l'intégration chromosomique d'au moins les gènes *araA, araB* et *araD* et *XylA* dans une souche de levure du type *Saccharomyces* qui, après culture selon la méthode de « Sequential Batch Repeat » telle que décrite dans WO 2009/112472 dans des milieux contenant 20g/L d'arabinose et 20g/L de xylose, lui confère la capacité à fermenter le glucose, le xylose et l'arabinose. Des mutations dans différents gènes (*SSY1, YJR154w, CEP3, GAL80, PMR1*) ont été mises en évidence.

Le document Madhavan et al. (2012, Critical Reviews in Biotechnology, 32(1), 22-48) répertorie les possibilités de construire des souches de *S. cerevisiae* capables de métaboliser les hexoses mais aussi les pentoses présents dans la lignocellulose.

Le document WO 2014/207087 décrit des microorganismes modifiés génétiquement pour produire des produits de fermentation à partir de pentoses, en particulier xylose et arabinose. Toutefois, les exemples n'illustrent que la métabolisation du xylose.

Il existe un besoin évident d'obtenir de nouvelles souches de levure capables de métaboliser l'arabinose, en plus de leur capacité à fermenter le glucose et le xylose y compris en présence d'inhibiteurs tels que l'acide acétique.

### DESCRIPTION DE L'INVENTION

La présente demande s'inscrit dans la recherche de nouvelles souches de levure aptes à métaboliser au moins l'arabinose, reposant sur la contribution des inventeurs en lien avec différents aspects :
- la mise en évidence du fait que l'introduction de la voie métabolique de l'arabinose est nécessaire mais non suffisante pour assurer la métabolisation efficace de l'arabinose ;
- la mise en évidence de la possibilité de sélectionner directement des transformants ayant intégré la voie de métabolisation de l'arabinose sur un milieu contenant de l'arabinose comme seule source de carbone ;
- la mise en évidence de la nécessité d'un contrôle étroit de la production de polyol pour assurer la fermentation efficace de l'arabinose ;
- la mise en évidence de la possibilité de faire coexister, de manière stable, les voies de fermentation du xylose et de l'arabinose ;
- la mise au point d'un protocole de sélection de souches de levure présentant une capacité améliorée à fermenter à la fois l'arabinose, le xylose et le glucose, y compris en présence d'inhibiteurs de la fermentation de ces deux derniers sucres à savoir l'acide acétique.

Selon un premier aspect, la présente invention concerne un procédé d'obtention et de sélection de souches de levure capables de fermenter l'arabinose, le glucose et le xylose en présence d'acide acétique sous forme non dissociée comprenant :
- l'intégration chromosomique des gènes *araA, araB* et *araD,* encore plus avantageusement des gènes *araA* de *B. licheniformis, araB* et *araD d'E. coli,* dans une souche de levure capable de fermenter le xylose en présence d'acide acétique sous forme non dissociée et présentant au moins un gène *GRE3* inactivé ou délété ;
- la mise en culture directe des souches transformées dans un milieu contenant de l'arabinose comme seule source de carbone pour la sélection des souches aptes à métaboliser ledit arabinose ;
- optionnellement la sélection des souches transformées et ayant métabolisé l'arabinose pour leur activité aldose réductase inférieure à 0,002 U / g de protéine, voire inférieure ou égale à 0,0005 U / g de protéine.

Dans le cadre de l'invention, on entend par « souche de levure » une population de levures rigoureusement identique du point de vue génétique. Cela englobe aussi bien les souches dites de laboratoire que les souches dites industrielles.

Avantageusement, la souche de levure mise en œuvre dans le cadre de ce procédé est choisie parmi *Saccharomyces, Schizosaccharomyces, Pichia, Yarrowia, Paffia, Kluyveromyces, Candida, Talaromyces, Brettanomyces, Pachysolen, Hansenula, Kloeckera, Schwanniomyces* et *Debaryomyces,* avantageusement une souche de *Saccharomyces cerevisiae.* Ces levures sont connues pour ne pas être capables naturellement de métaboliser l'arabinose ou à un niveau très faible, non exploitable industriellement. Elles sont par ailleurs avantageusement choisies pour leur capacité à réaliser la fermentation anaérobie, encore plus avantageusement la fermentation alcoolique anaérobie.

Selon l'invention, le procédé est mis en œuvre sur une souche apte à fermenter le xylose en présence d'acide acétique sous forme non dissociée. Comme largement décrit dans l'art antérieur, la capacité à métaboliser le xylose peut résulter de l'introduction d'un gène codant une xylose isomérase, par exemple provenant de *Clostridium phytofermentans,* et/ou une xylulokinase.

De manière avantageuse, il s'agit des souches suivantes :
- la souche déposée à la CNCM le 5.10.2011 sous le numéro I-4538,
- la souche déposée à la CNCM en date du 16.05.2013 sous le numéro I-4749 ;
- la souche déposée à la CNCM en date du 12.12.2013 sous le numéro I-4829 ;
- la souche déposée à la CNCM en date du 9.04.2015 sous le numéro I-4966 ;
encore plus avantageusement la souche déposée à la CNCM en date du 29 Janvier 2015 sous le numéro I-4953.

Dans le cadre de l'invention, une souche de levure apte à métaboliser l'arabinose est une souche capable de consommer ou d'utiliser l'arabinose, avantageusement le L-arabinose, présent dans son milieu de culture. Ainsi et notamment en fonction des conditions de culture, une souche de levure peut utiliser l'arabinose pour la production de sa biomasse et/ou la génération de produits de fermentation. Dans le cadre de la présente demande, la fermentation de l'arabinose s'entend comme la métabolisation de l'arabinose ayant lieu en hypoxie et/ou en anaérobiose, c'est-à-dire en conditions de faible disponibilité (typiquement moins de 20%) ou d'absence totale d'oxygène.

Au sens de l'invention, le terme « métaboliser » peut donc viser aussi bien la capacité de la souche de levure à utiliser l'arabinose pour assurer sa croissance que sa capacité à fermenter l'arabinose en divers produits de fermentation tels que des dérivés hydroxylés, dont l'éthanol ou l'isobutanol, et/ou carboxylés, dont les acides organiques.

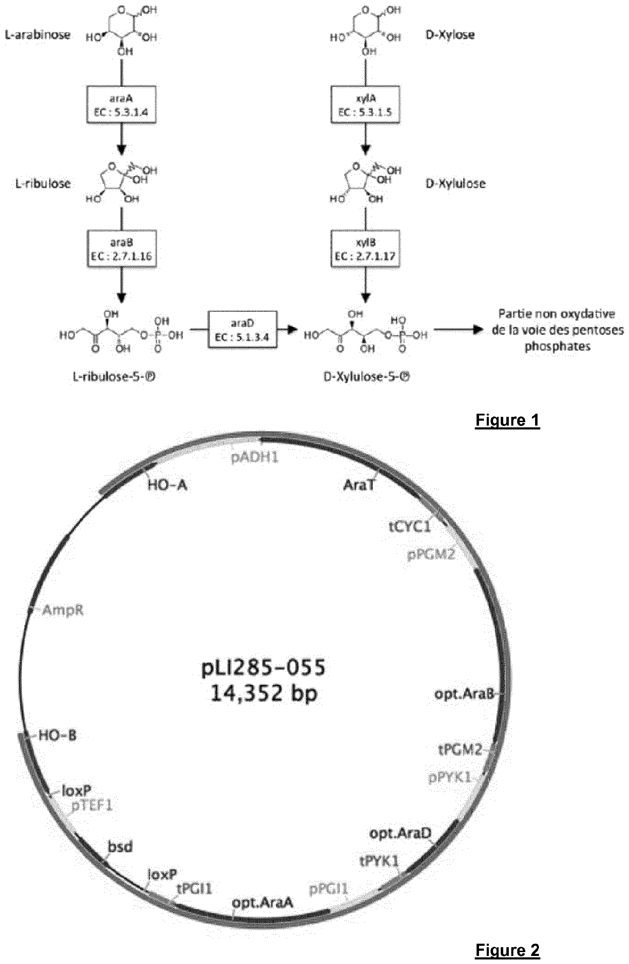

Selon un mode de réalisation particulier, il est fait référence à sa capacité à convertir le L-arabinose en L-ribulose et/ou L-ribulose-5-phosphate et/ou en D-xylulose-5-phosphate et/ou en un produit de fermentation tel que l'éthanol. Selon un mode de réalisation avantageux et comme décrit à la Figure 1, le L-arabinose est converti en L-ribulose sous l'action d'une arabinose isomérase (araA ; EC : 5.3.1.4). Le L-ribulose peut lui-même être converti en L-ribulose-5-phosphate sous l'action d'une ribulokinase (araB ; EC : 2.7.1.16). Le L-ribulose-5-phosphate peut alors être transformé en D-xylulose-5-phosphate sous l'action d'une ribulose 5 phosphate épimérase (araD ; EC : 5.1.3.4). De manière avantageuse, le D-xylulose-5-phosphate est alors pris en charge par la partie non oxydative de la voie des pentoses phosphates et peut aboutir à la production d'éthanol.

Dans une première étape du procédé selon l'invention, le ou les gènes permettant la métabolisation de l'arabinose sont introduits dans la souche de levure, qui est alors appelée transformant.

Dans la suite de l'exposé, les termes « gène » ou « séquence » visent une séquence d'acide nucléique, comprenant une séquence codante (codant notamment une enzyme de la voie d'intérêt), éventuellement flanquée de séquences régulatrices, en particulier un promoteur ou un terminateur. Une séquence codante peut être optimisée, c'est-à-dire modifiée pour intégrer les codons préférentiels de l'hôte, en l'occurrence la levure, dans lequel cette séquence est à exprimer.

Les gènes codant la voie de métabolisation de l'arabinose sont des gènes dits exogènes ou hétérologues, qui peuvent aussi bien être de nature synthétique ou issus d'autres organismes (ou sources). Avantageusement, ils proviennent de microorganismes aptes à métaboliser l'arabinose, avantageusement les hémi-ascomycètes tels que *Scheffersomyces stipitis,* les basidiomycètes, les champignons filamenteux ou les bactéries telles que *Erwinia chrysanthemi, Thermoanaerobacterium saccharolyticum, Escherichia coli, Zymomonas mobilis, Bacillus subtilis, Bacillus licheniformis* ou *Lactobacillus plantarum.* Selon un mode de réalisation particulier, ces gènes proviennent de la bactérie *Bacillus licheniformis* et/ou *Escherichia coli.* Selon un autre mode de réalisation privilégié, ces gènes correspondent au gène *araA* de *B. licheniformis,* au gène *araB d'E. coli* et au gène *araD d'E. coli,* tels que décrits par Widemann et Boles (2008, Applied and Environmental Microbiology 74, 2043-2050 ; WO 2008/122354).

Les techniques permettant l'introduction d'ADN dans un hôte (ou transformation) sont bien connues de l'homme du métier et comprennent notamment la perméabilisation des membranes par application d'un champ électrique (électroporation), par voie thermique (application d'un choc thermique) ou par voie chimique, par exemple à l'aide d'acétate de Lithium.

Les gènes introduits peuvent être intégrés dans le génome de l'hôte, notamment par recombinaison homologue ou intégration chromosomique, avantageusement à l'aide de cassettes intégratives, ou exprimés de manière extrachromosomique à l'aide de plasmides ou de vecteurs. Différents types de plasmides, avantageusement autoréplicatifs, sont bien connus de l'homme du métier, qui diffèrent notamment par leur origine de réplication, leur promoteur (inductible ou constitutif), leur marqueur (par exemple une résistance à un antibiotique ou la capacité à croitre dans un milieu sélectif) et le nombre de copies par cellule.

Les gènes codant la voie de métabolisation de l'arabinose sont intégrés au niveau chromosomique, avantageusement au niveau du locus HO de la souche de levure, encore plus avantageusement au niveau de tous les loci HO de la souche de levure.

De manière avantageuse, la cassette portant ce(s) gène(s) et servant à leur incorporation ou intégration dans la souche de levure est dépourvue de marqueur, notamment de résistance à un antibiotique.

Selon un autre mode de réalisation préféré, la cassette portant ce(s) gène(s) ne porte pas de gène codant un transporteur de l'arabinose, notamment le gène *araT.*

La bonne introduction du ou des gènes peut aisément être vérifiée par les techniques connues de l'homme du métier, par exemple à l'aide du marqueur éventuellement porté par la cassette d'expression, ou de manière préférée en réalisant une PCR à l'aide d'amorces visant le(s) gène(s) introduit(s). En outre, cette même technique de PCR peut être utilisée pour vérifier l'intégration du ou des gènes au locus visé, notamment en utilisant des amorces visant ce locus.

Dans une étape ultérieure du procédé selon l'invention, une souche d'intérêt ayant incorporé et exprimant efficacement la voie de métabolisation de l'arabinose est sélectionnée pour sa capacité à métaboliser l'arabinose présent, comme seule source de carbone, dans le milieu de culture.

De manière caractéristique selon l'invention, la sélection des transformants se fait directement sur un milieu contenant de l'arabinose comme seule source de carbone. En d'autres termes, la capacité d'une souche de levure à métaboliser l'arabinose est testée par culture de ladite souche sur un milieu contenant, comme seule source de carbone, ledit arabinose. Ceci exclut toute induction préalable, en particulier une culture préalable en présence de galactose ou l'ajout de galactose dans ce milieu, ou une présélection des transformants sur un milieu contenant une autre source de carbone comme par exemple le glucose.

Dans le cadre de l'invention, un milieu de culture ou de croissance désigne un milieu comprenant les ingrédients nécessaires à la multiplication des levures en présence. Il s'agit avantageusement d'un milieu complet, adapté à la croissance des levures et pouvant contenir des ingrédients classiques tels que des sels, des agents tampons, de l'extrait de levure ou toute autre source d'azote métabolisable par la levure, des vitamines.... Dans le cadre de l'invention, on entend par « milieu synthétique » un milieu dont la composition chimique est connue.

De manière adaptée, les conditions de culture mises en œuvre sont des conditions de culture favorables à la croissance des levures, en particulier :
- un milieu de culture à pH acide, avantageusement compris entre 4 et 6, voire 4,5 et 5,5, encore plus avantageusement égal à 5 ou 5,4 ;
- une température comprise entre 28 et 37°C, voire entre 30 et 35°C, avantageusement égale à 32°C ;
- un temps de croissance pouvant aller de 24 heures à plusieurs jours, par exemple 72 heures.

De manière avantageuse, cette croissance est réalisée en milieu solide, ce qui permet d'isoler les souches effectivement aptes à métaboliser l'arabinose. Selon un autre mode de réalisation avantageux, la sélection des souches d'intérêt est réalisée grâce à des cultures en boîte de Petri. De manière connue, les milieux solides contiennent de l'agar, avantageusement à hauteur de 15 à 20 g/L.

Selon un premier mode de réalisation, une souche de levure apte à métaboliser l'arabinose est sélectionnée par croissance aérobie de cette souche dans un milieu de croissance contenant, comme seule source de carbone, de l'arabinose.

Un milieu adapté à cette croissance aérobie est le milieu synthétique YNB Difco^{®}, dont la composition exacte est donnée dans les exemples de réalisation, comprenant de l'arabinose comme seule source de carbone, avantageusement à hauteur de 10 g/L, tel que le milieu appelé YNB-Ara ci-après.

Alternativement, une souche de levure apte à métaboliser l'arabinose peut être sélectionnée par croissance en anaérobiose ou en hypoxie dans un milieu de croissance contenant, comme seule source de carbone, de l'arabinose.

Un milieu adapté à cette croissance en anaérobiose ou en hypoxie est par exemple le milieu synthétique YF, dont la composition exacte est donnée dans les exemples de réalisation, comprenant de l'arabinose comme seule source de carbone, avantageusement à hauteur de 70 g/L, tel que le milieu appelé YF-ara ci-après.

Avantageusement, ce milieu est adapté pour permettre une croissance en milieu solide, par exemple par ajout d'agar, permettant ainsi d'isoler directement les souches d'intérêt.

Selon un mode de réalisation alternatif, les souches de levure dans lesquelles a été introduite la voie de métabolisation de l'arabinose sont isolées par culture en milieu solide, avantageusement sur un milieu sélectif tel que le milieu YNB-Ara décrit ci-dessus, puis sélectionnées en milieu liquide, en anaérobiose ou en hypoxie, dans un milieu adapté tel que le milieu YF-ara décrit ci-dessus. Ces cultures liquides peuvent être réalisées dans des microplaques de type Deep Weel ou en fioles, sous faible agitation, par exemple égale à 100 rpm, ou sans agitation et dans des conditions réduites d'apport en oxygène (sous O₂ limité ou en anaérobie).

Dans ces conditions, la capacité des souches de levure à métaboliser l'arabinose peut être évaluée par :
- leur croissance, notamment par le suivi de la densité optique (DO) du milieu de culture, avantageusement mesurée à 600 nm ; et/ou
- la fermentation de l'arabinose, notamment par le suivi de la concentration de l'arabinose présent dans le milieu de culture, par exemple par HPLC, ou l'évaluation de la perte de masse directement corrélée à la production de CO₂ qui est stoechiométrique avec celle de l'éthanol.

A noter qu'à la connaissance du Demandeur, il est rapporté pour la première fois la possibilité de sélectionner des souches de levure aptes à métaboliser l'arabinose directement par croissance sur un milieu contenant de l'arabinose comme seule source de carbone. Bien au contraire, il est considéré que l'homme du métier aurait été dissuadé de mettre en œuvre une telle approche au vu de l'enseignement de Wisselink et al. (2007; Appl Environ Microbiol 73, 4881-4891 ; WO 2008/041840) ayant rapporté qu'une telle sélection directe ne fonctionnait pas.

Selon un autre mode de réalisation avantageux, les souches de levure ainsi obtenues peuvent en outre être testées, en parallèle ou de manière subséquente, dans les conditions suivantes :
- sur un milieu de croissance contenant du glucose comme seule source de carbone, par exemple sur un milieu YF comme décrit ci-dessus mais contenant 150 g/L de glucose, et dans les conditions de culture mentionnées ci-dessus. Cette étape permet de vérifier la validité des souches sélectionnées, notamment leur capacité à fermenter le glucose. A noter que un ou plusieurs passages sur milieu glucose peuvent être réalisés avant de « repasser » les souches sélectionnées sur un milieu contenant de l'arabinose comme seule source de carbone (comme décrit ci-dessus) et ce pour vérifier que le phénotype [ara+] est stable et conservé ;
- un milieu de croissance contenant du xylose comme seule source de carbone, par exemple sur un milieu YF comme décrit ci-dessus mais contenant 70 g/L de xylose (milieu YF-xylose dans les exemples de réalisation), et dans les conditions de culture mentionnées ci-dessus. Cette étape permet de vérifier que les souches sélectionnées ont conservé leur capacité à fermenter le xylose et présente un intérêt lorsque la souche de levure sur laquelle est mis en œuvre le procédé de l'invention possède une capacité à fermenter le xylose.

En outre et dans le cadre de l'invention, il a été mis en évidence qu'un critère important pour la sélection de souches d'intérêt pour la métabolisation de l'arabinose était leur niveau de production de polyols.

De manière connue, les polyols comme le xylitol possèdent un effet inhibiteur sur l'activité xylose isomérase (xylA) (Kovalevsky et al., 2012, Acta Crystallogr. D Biol. Crystallogr. 68, 1201-1206). Dans le cadre de la présente invention, il a été mis en évidence que les polyols étaient également susceptibles d'inhiber l'activité arabinose isomérase (araA ; Figure 1), et ainsi de diminuer les performances, en termes de métabolisation de l'arabinose, d'une souche sélectionnée à l'aide du procédé selon l'invention.

L'arabitol est susceptible d'être généré à partir d'arabinose sous l'action d'aldose réductase(s), de la même manière que le xylitol est généré à partir de xylose. A noter que de manière connue, le gène *GRE3* code l'aldose réductase principale chez *S. cerevisiae* mais que, même lorsque celui-ci est délété ou inactivé, il peut persister une activité aldose réductase. Par ailleurs, la présence dans les souches de levure d'une activité xylitol déshydrogénase permet potentiellement d'éliminer le xylitol.

Ainsi et selon cette hypothèse, une souche sélectionnée particulièrement d'intérêt présente :
- une forte activité polyol déshydrogénase, avantageusement supérieure ou égale à 0,001 U / g de protéine, encore plus avantageusement supérieure ou égale à 0,002 U / g de protéine; et/ou
- une faible activité aldose réductase.

En pratique et dans le cadre de l'invention, il a été mis en évidence qu'une souche d'intérêt pouvait être sélectionnée en raison de son activité aldose réductase faible voire nulle. Avantageusement, cette activité est inférieure à 0,005 U / g de protéine, voire 0,004, 0,003 voire même 0,002 U / g de protéine. De manière encore préférée, elle est inférieure ou égale à 0,0015 U / g de protéine, voire inférieure ou égale à 0,001 U / g de protéine, encore plus avantageusement inférieure ou égale à 0,0005 U / g de protéine.

Un protocole détaillé pour la mesure de l'activité aldose réductase est décrit dans la partie « Exemples de réalisation ».

En outre, la souche de levure mise en œuvre dans le cadre du procédé selon l'invention présente un ou des gènes *GRE3* délété(s) ou inactivé(s).

Selon un autre mode de réalisation privilégié, une souche de levure mise en œuvre dans le cadre de la présente invention présente au moins une copie surnuméraire du gène *HAA1* codant le régulateur transcriptionnel Haa1p. De manière connue, celui-ci est capable de conférer aux souches de levure, en particulier *S. cerevisiae,* une capacité à résister à l'acide acétique et permet donc d'améliorer leur croissance dans un milieu contenant des inhibiteurs de la fermentation de type acide organique, tel que l'acide acétique. En outre, le demandeur a mis en évidence que la surexpression de ce gène conférait une résistance à d'autres inhibiteurs de type composés phénoliques, en particulier la vanilline.

De manière avantageuse, l'expression du gène surnuméraire codant Haa1p est placée sous le contrôle d'un promoteur hétérologue, par exemple le promoteur pPGK1. Cette copie surnuméraire du gène *HAA1* peut coder la protéine native ou une forme mutée de celle-ci, par exemple la version constitutivement active *HAA1_{S135F}* décrite par Swinnen et al. (2017, Microbial Cell Factories 16 : 7).

Selon un mode de réalisation préféré, la copie additionnelle du gène *HAA1* est intégrée au niveau chromosomique, encore plus avantageusement par insertion au niveau du gène *GRE3.* Il en résulte deux effets avantageux dans le cadre de l'invention, à savoir :
- l'inactivation du gène *GRE3* codant une activité aldose réductase ;
- la surexpression du gène *HAA1* conférant une plus grande résistance des souches de levure à l'acide acétique mais également à la vanilline.

La seconde étape du procédé selon l'invention, correspondant à l'étape de sélection des souches de levure d'intérêt. Selon un mode de réalisation particulier, l'aptitude à métaboliser l'arabinose présent dans le milieu de culture est d'abord évaluée puis les souches sélectionnées sont testées pour leur activité aldose réductase.

L'intérêt des souches ainsi sélectionnées, notamment leur capacité à fermenter l'arabinose, mais également le glucose et le xylose, peut alors être confirmé par l'évaluation de leur performances sur des milieux synthétiques contenant les différents sucres en mélange, à savoir l'arabinose, le glucose et le xylose. Afin de mimer les conditions réelles de fermentation, ces milieux contiennent également de l'acide acétique, avantageusement à hauteur de 1 à 10 g/L, connu pour être un inhibiteur de la fermentation du glucose et du xylose.

De tels milieux sont par exemple les milieux notés YFP ou YFCF dont la composition est donnée ci-dessous :

### Milieu YFCF :

- 10 g/L d'extrait de levure ;
- 10 g/L de bacto-peptone ;
- 63 g/L de glucose;
- 28 g/L de xylose;
- 28 g/L d'arabinose;
- 4 g/L d'acide acétique (quantité introduite dans le milieu de culture à pH 5).

### Milieu YFP :

- 10 g/L d'extrait de levure ;
- 10 g/L de bacto-peptone ;
- 63 g/L de glucose;
- 52 g/L de xylose;
- 6,1 g/L d'arabinose;
- 4 g/L d'acide acétique (quantité introduite dans le milieu de culture à pH 5).

Par ailleurs, des conditions standards de culture, favorables à la production d'éthanol chez les levures, sont :
- un pH acide et stable, avantageusement compris entre 4 et 6, par exemple égal à 5 ;
- une température comprise entre 28 et 37°C, voire entre 30 et 35°C, avantageusement égale à 30°C ;
- une faible agitation, par exemple égale à 100 rpm ;
- des conditions réduites d'apport en oxygène (sous O₂ limité). En pratique, la culture peut être réalisée dans une fiole obturée à l'aide d'un bouchon réduisant l'apport d'O₂ dans le milieu tout en permettant l'évacuation du CO₂ produit ;
- un ensemencement avec un inoculum de 0,25 g/kg eq MS de la souche propagée à saturation sur YPG à 24 heures ;
- un temps de croissance d'au moins 24 heures, par exemple de 72 heures.

Dans le cadre de l'invention, il a été mis en évidence des souches sélectionnées à l'aide de ce procédé présentant :
- la capacité à utiliser l'arabinose comme seule source de carbone pour assurer la production de leur biomasse ;
- la capacité à fermenter l'arabinose ;
- la capacité à fermenter le glucose et le xylose, y compris en présence d'acide acétique sous forme non dissociée.

Dans les conditions décrites ci-dessus, il a été observé après 72 heures de fermentation :
- la consommation de 95% voire de l'intégralité du glucose et du xylose présents dans le milieu de culture, convertis soit en biomasse, soit en éthanol et CO₂ ;
- la consommation de plus de 50%, voire 60%, 70% ou même 80% de l'arabinose présent dans le milieu de culture.

Ainsi, la présente demande rapporte une souche de levure susceptible d'être obtenue à l'aide du procédé décrit, capable de fermenter au moins 50%, voire 60%, 70% ou même 80% de l'arabinose après 72 heures de fermentation dans un milieu comprenant du glucose (avantageusement à hauteur de 1 à 100 g/L, par exemple 63 g/L), du xylose (avantageusement à hauteur de 1 à 100 g/L, par exemple 28 ou 52 g/L), de l'arabinose (avantageusement à hauteur de 1 à 100 g/L, par exemple 6,1 ou 28 g/L) et de l'acide acétique (avantageusement à hauteur de 1 à 10 g/L, par exemple 4 g/L). De manière préférée, une telle souche de levure est capable de fermenter dans le même temps au moins 90% voire 95% du glucose et du xylose également présents.

Une telle souche présente en outre avantageusement au moins l'une des caractéristiques suivantes, voire toutes ces caractéristiques :
- au moins une copie d'un gène *araA,* de préférence issu de *B. licheniformis,* avantageusement intégré au niveau chromosomique, encore plus avantageusement au niveau d'au moins un locus HO ;
- au moins une copie d'un gène *araB,* de préférence issu d'E. *coli,* avantageusement intégré au niveau chromosomique, encore plus avantageusement au niveau d'au moins un locus HO ;
- au moins une copie d'un gène *araD,* de préférence issu d'E. *coli,* avantageusement intégré au niveau chromosomique, encore plus avantageusement au niveau d'au moins un locus HO ;
- au moins une copie d'un gène exogène codant une xylose isomérase, avantageusement de *Clostridium phytofermentans ;*
- au moins une copie surnuméraire du gène *GAL2* codant un transporteur des hexoses également capable d'assurer la capture du xylose. Selon un autre mode de réalisation, ladite souche comprend au moins deux copies surnuméraires du gène *GAL2.* Celui-ci peut être placé sous le contrôle d'un promoteur fort et constitutif de type pADH1 ;
- la suppression de l'activité aldose réductase codée par *GRE3,* avantageusement par insertion du gène *HAA1* au niveau du locus *GRE3 ;*
- la surproduction de la xylulokinase (XKS1), notamment par modification du promoteur ou introduction de copies surnuméraires ;
- l'expression ou la surproduction de la voie des pentoses phosphates (RPE1, RKI1, TKL1, TAL1, ...) ;
- l'absence d'activité xylose réductase (XR).

Selon un mode de réalisation particulier, une telle souche n'est pas mutée au niveau des gènes suivants : *SSY1, YJR154w, CEP3, GAL80* et/ou *PMR1.* Selon un autre mode de réalisation particulier, la souche ne porte pas les mutations suivantes : G1363T dans le gène *SSY1,* A512T dans le gène *YJR154w,* A1186G dans le gène *CEP3,* A436C dans le gène *GAL80,* A113G dans le gène *PMR1.*

Une souche selon l'invention est la souche déposée auprès de la CNCM (Collection Nationale de Cultures de Microorganismes, Institut Pasteur, 25 rue du Docteur Roux, 75724 Paris Cedex 15) en date du 19 Mai 2016, sous le numéro I-5085.

Selon un autre aspect, la demande concerne un procédé d'obtention et/ou sélection de souches de levure présentant une capacité améliorée à fermenter l'arabinose, le xylose et le glucose, y compris en présence d'inhibiteurs de la fermentation à savoir de l'acide acétique sous forme non dissociée, se traduisant notamment par une augmentation de la production d'éthanol et une plus grande consommation de l'arabinose présent dans le milieu.

Ainsi, l'invention concerne également un procédé d'obtention et de sélection d'une souche de levure présentant une capacité améliorée à fermenter l'arabinose, le glucose et le xylose en présence d'acide acétique sous forme non dissociée, dans lequel une souche de levure présentant une capacité à fermenter l'arabinose, le glucose et le xylose en présence d'acide acétique sous forme non dissociée et obtenue à l'aide du procédé décrit ci-dessus, avantageusement la souche déposée I-5085, est cultivée successivement dans les conditions suivantes :
- une culture en anaérobiose ou en hypoxie dans un milieu contenant, comme seules sources de carbone, de l'arabinose et du xylose en concentrations limitantes pour la production de biomasse, avantageusement à hauteur de 5 g/L et 4 g/L, respectivement ; puis
- deux cultures successives en anaérobiose ou en hypoxie et en présence d'acide acétique sous forme non dissociée, l'une dans un milieu contenant du glucose comme seule source de carbone et l'autre dans un milieu contenant du xylose comme seule source de carbone ;
- éventuellement une culture en aérobiose dans un milieu minimum contenant comme seule source de carbone une source de carbone respiratoire stricte, avantageusement du glycérol.

Un tel procédé consiste donc à réaliser une évolution dirigée des souches de levure mises en œuvre. Sans vouloir être liés à une quelconque théorie, la pression de sélection exercée par les cultures successives dans les milieux de croissance définis ci-après permet de sélectionner une souche ayant acquis les traits phénotypiques nécessaires à l'augmentation de sa capacité à fermenter l'arabinose tout en conservant sa capacité à fermenter le xylose et le glucose, y compris en présence d'acide acétique sous forme non dissociée.

Ainsi, le procédé selon l'invention permet, à partir d'une souche isolée ou d'un mélange de souches, de sélectionner une souche présentant un avantage sélectif en termes de croissance sur un milieu contenant ces trois sucres et l'acide acétique sous forme non dissociée.

Ce procédé, également objet de la présente demande, peut être mis en œuvre sur une souche isolée, en particulier sur la souche déposée auprès de la CNCM (Collection Nationale de Cultures de Microorganismes, Institut Pasteur, 25 rue du Docteur Roux, 75724 Paris Cedex 15) en date du 19 Mai 2016, sous le numéro I-5085.

Selon ce procédé, la souche de levure ou un éventuel mélange de souches est cultivé successivement dans au moins trois milieux de croissance. Comme déjà dit, les expressions « milieu de croissance » ou « milieu de culture » sont utilisées indifféremment pour désigner un milieu comprenant les ingrédients nécessaires à la multiplication des levures en présence.

Le premier milieu de croissance, avantageusement liquide, se caractérise en ce qu'il comprend, comme seules sources de carbone, les pentoses arabinose et xylose.

A noter qu'il est traditionnellement convenu que lorsqu'une population de levures dispose de deux sucres, elle n'en utilise généralement qu'un seul pour faire sa biomasse. Une fois ce premier sucre consommé, elle peut éventuellement utiliser le second. Ainsi, il y avait un risque réel concernant la stabilité du phénotype de fermentation des deux pentoses d'intérêt, à savoir le xylose et l'arabinose.

De manière caractéristique selon l'invention, ce premier milieu comprend une concentration en pentoses permettant de couvrir ensemble 100%, avantageusement chacun 50%, des besoins en carbone des souches de levure. Selon un autre mode de réalisation particulier, l'arabinose et le xylose sont présents à des concentrations équivalentes, par exemple à raison de 5 g/L pour l'arabinose et de 4 g/L pour le xylose.

Selon une autre caractéristique privilégiée, il s'agit d'un milieu synthétique dont la composition exacte est maîtrisée, avantageusement un milieu dont la composition chimique est entièrement déterminée. Un tel milieu est par exemple le milieu nommé « GO xylose arabinose » dont la composition précise est donnée dans les exemples de réalisation. Il est à noter que dans un tel milieu, ce n'est pas la source d'azote qui est limitante pour la production de biomasse (10 g/L de (NH₄)₂PO₄) mais les quantités en sources de carbone, en l'occurrence le xylose (4 g/L) et l'arabinose (5 g/L). Ainsi, un tel milieu favorise la croissance et la multiplication des souches de levure qui utilisent les deux sucres.

L'étape suivante vise à anticiper un éventuel risque de perte de tolérance vis-à-vis des inhibiteurs, en particulier de l'acide acétique sous forme non dissociée, des souches sélectionnées.

Ainsi et dans cette étape, deux cultures anaérobies successives sont mises en œuvre :
- l'une dans un milieu de croissance contenant du glucose comme seule source de carbone ; et
- l'autre dans un milieu de croissance contenant du xylose comme seule source de carbone.

L'ordre de passage sur ces deux milieux de culture peut éventuellement être inversé (milieu xylose puis glucose).

De manière avantageuse, la concentration en glucose ou en xylose du milieu de croissance est celle couramment mise en œuvre lorsque celui-ci est utilisé comme seule source de carbone, à savoir comprise entre 5 et 200 g/L dans le cas d'un milieu liquide (5 et 200 g/kg dans le cas d'un milieu solide), par exemple égale à 150 g/L pour le glucose et 70 g/L pour le xylose.

Ledit milieu comprend en outre l'acide organique susceptible d'inhiber la fermentation de ces deux sucres, à savoirde l'acide acétique sous forme non dissociée.

A noter que de manière connue, seule la forme non dissociée ou non ionisée de tels acides présente une capacité d'inhibition. Dans le cadre de l'invention, on entend par « forme non ionisée ou non dissociée » d'un acide carboxylique sa forme protonée. En pratique, la forme de tels acides organiques dépend du pH du milieu dans lequel ils sont incorporés. A un pH supérieur au pKa de l'acide, celui-ci se trouve majoritairement sous forme dissociée ou d'ions COO⁻. Au contraire et à un pH inférieur, la forme majoritaire est la forme non dissociée ou non ionisée (COOH). Dans la suite de la description, il est fait référence à l'acide acétique introduit dans le milieu, englobant formes dissociée et non dissociée en fonction du pH dudit milieu.

De manière avantageuse, la concentration en acide acétique sous forme non dissociée du milieu de croissance est comprise entre 0,5 et 5 g/L en milieu liquide (équivalent à 0,5 et 5g/kg en milieu solide), avantageusement entre 1,3 et 2,6 g/L. En pratique et à titre d'exemple, cette dernière fourchette correspond à une concentration en acide acétique ajoutée à un milieu de croissance à pH 5 comprise entre 3 et 6 g/L, par exemple égale à 4 ou 5 g/L.

Outre ces deux ingrédients, ce milieu de croissance est avantageusement un milieu synthétique complet, adapté à la croissance des levures en anaérobiose ou en hypoxie, comme par exemple le milieu YF dont la composition exacte est donnée dans les exemples de réalisation.

Ainsi, des milieux adaptés pour la mise en œuvre de cette seconde étape du procédé selon l'invention sont par exemples les milieux appelés YF-glucose acide et YF-xylose acide, dont la composition est détaillée dans les exemples de réalisation.

Outre la composition de ces milieux de croissance, la culture de la souche de levure ou du mélange de souches dans ces première et deuxième étapes du procédé selon l'invention est avantageusement réalisée dans des conditions standard favorables à la croissance en anaérobiose ou en hypoxie des levures, en particulier de type *Saccharomyces,* et à leur activité de fermentation, à savoir :
- un pH acide, avantageusement compris entre 4 et 6, voire 4,5 et 5,5, encore plus avantageusement égal à 5 ou 5,4 ;
- une température comprise entre 28 et 37°C, voire entre 30 et 35°C, avantageusement égale à 32°C ;
- une culture sous faible agitation, par exemple égale à 100 rpm ;
- dans des conditions réduites d'apport en oxygène (sous O₂ limité). En pratique, la culture peut être réalisée dans une fiole obturée à l'aide d'un bouchon réduisant l'apport d'O₂ dans le milieu tout en permettant l'évacuation du CO₂ produit.

De manière générale, la culture est stoppée lorsque la source de carbone osidique a été totalement consommée. En pratique et de manière avantageuse, la culture est menée pendant au moins 24 heures, voire plusieurs jours, avantageusement jusqu'à 7 jours. Selon un mode de réalisation particulier, le procédé selon l'invention comprend en outre le passage des levures sur un quatrième milieu de croissance, avantageusement liquide, destiné à sélectionner les cellules capables de respirer, à savoir présentant des mitochondries fonctionnelles. En pratique, cette étape, qui peut être mise en œuvre à chaque cycle ou au moins une fois dans le procédé, permet de s'affranchir de l'apparition des « petites », dont le phénotype déficient respiratoire peut être désavantageux dans le cadre des procédés de production de levures industrielles.

De manière avantageuse, ce quatrième milieu est un milieu pauvre ou minimum, contenant comme seule source de carbone une source de carbone qui ne peut être utilisée que par les cellules qui ont conservé des mitochondries fonctionnelles. On parle dans ce cas de source de carbone respiratoire stricte, c'est à dire de source de carbone impliquant de manière systématique une oxydation mitochondriale et ne produisant pas d'éthanol. Il peut s'agir avantageusement du glycérol ou éventuellement de l'éthanol. En d'autres termes, un tel milieu est dépourvu de sucre fermentescible.

Avantageusement, la concentration en glycérol du milieu de croissance est celle couramment mise en œuvre lorsque celui-ci est utilisé comme seule source de carbone, à savoir comprise entre 5 et 50 g/L, avantageusement comprise entre 10 et 50 g/L, par exemple égale à 10 g/L de manière à obtenir une biomasse suffisante pour éventuellement inoculer le premier milieu de culture du cycle suivant.

Par définition, un milieu minimum contient, outre une source de carbone, une source d'azote, une source de potassium, une source de phosphore, une source de soufre, une source de magnésium, une source de calcium, une source de fer, une source d'oligoéléments et de l'eau.

Un milieu pouvant servir à l'élaboration de ce milieu de culture peut comprendre :
- une base, telle que « yeast nitrogen base » de DIFCO^{®}, avantageusement à hauteur de 3,4 g/L ;
- et éventuellement du sulfate d'ammonium, avantageusement à hauteur de 5 g/L.

Outre la composition spécifique de ce milieu de croissance, la culture de la souche de levure ou du mélange de souches est avantageusement réalisée dans des conditions standard favorables à la croissance des levures en aérobiose, à savoir :
- un pH acide, avantageusement compris entre 4 et 6, voire 4,5 et 5,5, par exemple égal à 5 ;
- une température comprise entre 28 et 37°C, voire entre 30 et 35°C, avantageusement égale à 32°C ;
- sous agitation moyenne, par exemple égale à 150 rpm ;
- en aérobiose. En pratique, la culture peut être réalisée dans une fiole à baffles obturée par un bouchon poreux qui permet l'apport d'O₂ dans le milieu.

Là encore, la culture est stoppée lorsque la source de carbone, avantageusement le glycérol, a été totalement consommée. En pratique et de manière avantageuse, la culture est menée pendant plusieurs heures, avantageusement 48 heures.

Cette succession de culture dans ces trois voire quatre milieux, et dans les conditions décrites ci-dessus, constitue un cycle.

En pratique un cycle de sélection selon l'invention peut donc se décliner comme suit :
- une première culture en anaérobiose ou hypoxie en présence de xylose et d'arabinose ;
- une seconde culture en anaérobiose ou hypoxie en présence de glucose et d'acide acétique ;
- une troisième culture en anaérobiose ou hypoxie en présence de xylose et d'acide acétique ;
- éventuellement une quatrième culture en aérobie et en présence de glycérol ;
   ou
- une première culture en anaérobiose ou hypoxie en présence de xylose et d'arabinose ;
- une seconde culture en anaérobiose ou hypoxie en présence de xylose et d'acide acétique ;
- une troisième culture en anaérobiose ou hypoxie en présence de glucose et d'acide acétique ;
- éventuellement une quatrième culture en aérobie et en présence de glycérol.

Selon l'invention, ces différentes cultures sont réalisées selon la méthode dite de « Single Batch Repeat », c'est-à-dire sans renouvellement des milieux de culture en présence.

Selon un mode de réalisation particulier, un tel cycle est répété au moins 2 fois (« Multiple Batch Repeat »), par exemple 2 fois.

Ce procédé a permis la sélection de souches de levure qui, lors de la fermentation sur un milieu proche des milieux naturels comprenant notamment de l'arabinose, du glucose, du xylose et de l'acide acétique, présentent une production d'éthanol améliorée (un titre alcoolique plus important) et une meilleure consommation de l'arabinose présent dans le milieu.

Selon un autre aspect, la présente demande rapporte une souche de levure susceptible d'être obtenue à l'aide du procédé décrit. Ainsi, il a pu être isolé des souches de levure qui, à l'issue de ce procédé de sélection, présentent une capacité de consommation de l'arabinose présent dans le milieu augmentée d'au moins 10%, voire 20%, 30%, 40%, 50%, 60%, 70%, voire même 80%. Ces performances sont avantageusement évaluées dans des conditions de fermentation comme décrites ci-dessus en relation avec le milieu YFCF et après 160 heures.

En effet et dans ces conditions, il a été observé une consommation de plus de 90%, voire 95% ou même 97,5% de l'arabinose présent dans le milieu de culture.

Ainsi, la présente demande rapporte une souche de levure susceptible d'être obtenue à l'aide du procédé décrit, capable de fermenter au moins 90%, voire 95%, ou même 97,5% de l'arabinose après 160 heures de fermentation dans un milieu comprenant du glucose (avantageusement à hauteur de 1 à 100 g/L, par exemple 63 g/L), du xylose (avantageusement à hauteur de 1 à 100 g/L, par exemple 28 g/L), de l'arabinose (avantageusement à hauteur de 1 à 100 g/L, par exemple 28 g/L) et de l'acide acétique (avantageusement à hauteur de 1 à 10 g/L, par exemple 4 g/L). De manière préférée, une telle souche de levure est capable de fermenter dans le même temps au moins 90% voire 95% voire même 100% du glucose et du xylose également présents.

Avantageusement, une telle souche présente au moins l'une des caractéristiques suivantes, voire toutes ces caractéristiques :
- au moins une copie d'un gène *araA,* de préférence issu de *B. licheniformis,* avantageusement intégré au niveau chromosomique, encore plus avantageusement au niveau de tous les loci HO ;
- au moins une copie d'un gène *araB,* de préférence issu d'E. *coli,* avantageusement intégré au niveau chromosomique, encore plus avantageusement au niveau de tous les loci HO ;
- au moins une copie d'un gène *araD,* de préférence issu d'E. *coli,* avantageusement intégré au niveau chromosomique, encore plus avantageusement au niveau de tous les loci HO ;
- au moins une copie d'un gène exogène codant une xylose isomérase, avantageusement de *Clostridium phytofermentans ;*
- au moins une copie surnuméraire du gène *GAL2* codant un transporteur des hexoses également capable d'assurer la capture du xylose. Selon un autre mode de réalisation, ladite souche comprend au moins deux copies surnuméraires du gène *GAL2.* Celui-ci peut être placé sous le contrôle d'un promoteur fort et constitutif de type pADH1 ;
- la suppression de l'activité aldose réductase codée par *GRE3,* avantageusement par insertion du gène *HAA1* au niveau du locus *GRE3 ;*
- la surproduction de la xylulokinase (XKS1), notamment par modification du promoteur ou introduction de copies surnuméraires ;
- l'expression ou la surproduction de la voie des pentoses phosphates (RPE1, RKI1, TKL1, TAL1, ...) ;
- l'absence d'activité xylose réductase (XR).

Selon un autre mode de réalisation avantageux, une telle souche présente au moins l'une des caractéristiques suivantes, avantageusement les 2 :
- une forte activité polyol déshydrogénase, avantageusement supérieure ou égale à 0,001 U / g de protéine, encore plus avantageusement supérieure ou égale à 0,002 U / g de protéine ;
- une activité aldose réductase inférieure à 0,005 U / g de protéine, voire 0,004, 0,003 voire même 0,002 U / g de protéine, préférentiellement inférieure ou égale à 0,0015 U / g de protéine, voire inférieure ou égale à 0,001 U / g de protéine, encore plus préférentiellement inférieure ou égale à 0,0005 U / g de protéine.

Selon un mode de réalisation particulier, une telle souche n'est pas mutée au niveau des gènes suivants : *SSY1, YJR154w, CEP3, GAL80* et/ou *PMR1.* Selon un autre mode de réalisation particulier, la souche ne porte pas les mutations suivantes : G1363T dans le gène *SSY1,* A512T dans le gène *YJR154w,* A1186G dans le gène *CEP3,* A436C dans le gène *GAL80,* A113G dans le gène *PMR1.*

Une souche selon l'invention est la souche déposée auprès de la CNCM (Collection Nationale de Cultures de Microorganismes, Institut Pasteur, 25 rue du Docteur Roux, 75724 Paris Cedex 15) en date du 19 Mai 2016, sous le numéro I-5086.

Selon un mode de réalisation particulier, une telle souche possède au moins une copie surnuméraire du gène *HAA1,* avantageusement insérée au niveau du gène *GRE3.*

Selon un autre aspect, l'invention concerne l'utilisation de souches telles que définies ci-dessus pour la fermentation d'un matériau, contenant avantageusement de l'arabinose et/ou du xylose et/ou du glucose, et/ou pour la production d'éthanol.

Selon un mode de réalisation particulier, le matériau est un matériau lignocellulosique. Un tel matériau contient typiquement :
- des pentoses, en particulier D-xylose et L-arabinose ;
- des hexoses, en particulier du D-mannose, du D-galactose, du L-rhamnose et du D-glucose ;
- des acides uroniques.

Selon un aspect particulier, l'invention a trait à un procédé de production de produits de fermentation ou d'éthanol comprenant les étapes suivantes :
- Incubation d'un matériau ou milieu contenant de l'arabinose et/ou du xylose et/ou du glucose, avantageusement de l'arabinose, du xylose, du glucose et de l'acide acétique, avec une souche telle que définie ci-dessus ;
- Fermentation en condition anérobie ou semi-anaérobie (hypoxie) ;
- Récupération du ou des produits de fermentation, ou de l'éthanol.

Selon un mode de réalisation particulier, le matériau ou milieu est de l'hémicellulose ou des « corn fibers ».

La présente invention va être illustrée plus avant à l'aide des exemples de réalisation qui suivent, à l'appui des figures annexées. Toutefois, ceux-ci n'ont aucune portée limitative.

### LEGENDES DES FIGURES :

La figure 1 représente les voies métaboliques de conversion du L-arabinose et du D-xylose en D-xylulose-5-phosphate.
La figure 2 représente le plasmide pLI285-055 permettant l'intégration des gènes *araA*/*araB*/*araD* au niveau du locus HO de *Saccharomyces cerevisiae.*
La figure 3 représente les produits de PCR obtenus en utilisant les oligonucléotides du tableau 1 ci-dessous. La piste 1 correspond au marqueur de taille (1 kb DNA ladder), les pistes 2 à 9 correspondent aux produits de PCR obtenus en utilisant comme matrice l'ADN génomique des 8 transformants testés, la piste 10 à celui obtenu avec la souche non transformée. La piste 11 est le témoin négatif de la technique (eau) et la piste 12 est le témoin positif avec comme matrice le plasmide pLI285-055.
La figure 4 illustre la croissance des colonies sur un milieu YPG + blasticidine 50 mg/L (A) et sur un milieu YNB-Ara contenant 10 g/L d'arabinose (B).
La figure 5 illustre la croissance des clones sélectionnés, sur un milieu contenant de l'arabinose (A) ou du xylose (B) comme seule source de carbone.
La figure 6 représente l'évolution de la concentration (exprimée en g/kg) de différents constituants en fonction du temps de fermentation, sur un milieu YFCF (63g/kg de glucose, 28g/kg de xylose, 28g/kg de d'arabinose et 4g/kg d'acide acétique pH = 5), des clones 13 (A) et 126 (B) sélectionnés pour leur capacité à fermenter l'arabinose. Les valeurs représentent les valeurs moyennes issues de 3 expériences biologiquement indépendantes.
La figure 7 représente l'activité aldose réductase mesurée dans les différents fonds génétiques testés (I-4953 ; clone 13 ; clone 126). Les valeurs représentent les valeurs moyennes issues de 2 expériences biologiquement indépendantes.
La figure 8 représente la perte de masse observée après 48 heures de fermentation dans le clone 126 et ses transformants (transformés avec pADH1-GRE3).
La figure 9 représente (A) l'évolution de la concentration en éthanol (g/kg) en fonction du temps de fermentation sur un milieu YFCF de différents clones sélectionnés après une évolution dirigée réalisée à partir de la souche EG31 ; (B) la concentration en arabinose (g/kg) après 160 heures de fermentation sur milieu YFCF de ces mêmes clones et de la souche EG31.

### EXEMPLES DE REALISATION

### I/ Obtention d'une souche de Saccharomyces cerevisiae apte à fermenter l'arabinose :

### 1. Transformation par la voie arabinose :

La voie de fermentation de l'arabinose est représentée à la Figure 1.

La souche notée I-4953, déposée auprès de l'Institut Pasteur (Collection Nationale de Cultures de Microorganismes, 25 rue du Docteur Roux, 75724 Paris Cedex 15) sous le numéro CNCM I-4953 en date du 29 janvier 2015, a été transformée à l'aide du plasmide pLI285-055 (Figure 2), un dérivé du plasmide pHD22 portant une cassette d'expression de la voie arabinose permettant l'intégration des gènes *araA* de *Bacillus licheniformis* et *araB*/*araD d'Escherichia coli* au niveau du locus *HO* de cette souche de *S. cerevisiae.*

Cette souche est par ailleurs connue pour être capable de métaboliser le xylose en raison de la présence d'au moins une copie d'un gène exogène codant la xylose isomérase de *Clostridium phytofermentans.*

De plus, cette souche surexprime les gènes codant les enzymes de la partie non oxydative de la voie des pentoses phosphates, notamment les gènes *TAL1* et *TKL1.*

### 2. Validation de l'intégration chromosomique de la voie arabinose :

La bonne intégration des différents gènes qui codent les enzymes constituant la voie de métabolisation de l'arabinose, en particulier des gènes *ara A, B* et *D,* a été vérifiée par PCR en utilisant comme matrice l'ADN génomique des transformants et des oligonucléotides permettant d'amplifier un fragment de chacun des 3 ORF correspondants. Ces oligonucléotides, ainsi que leur séquence, sont indiqués dans le tableau 1 ci-dessous :

| Nom | Séquence | Taille attendue |
|---|---|---|
| araA-v-f-1 (SEQ ID NO : 1) | ATGATTCAAGCTAAGACCC | 350 pb |
| araA-v-r-1 (SEQ ID NO : 2) | ATGTCAATCTTGTCCCATGG | |
| araB-v-f-1 (SEQ ID NO : 3) | ATGGCTATTGCTATTGGTTT | 357 pb |
| araB-v-r-2 (SEQ ID NO : 4) | CCACAAAACGAACATAGCGT | |
| araD-v-f-1 (SEQ ID NO : 5) | ATGTTGGAAGACTTGAAGAG | 363 pb |
| araD-v-r-1 (SEQ ID NO : 6) | TAGAAGTAGTCAGCGTGGGT | |

Les produits de PCR obtenus ont été analysés sur un gel d'agarose à 0,8% après une migration d'une heure à 50 Volts dans du TAE 0,5x.

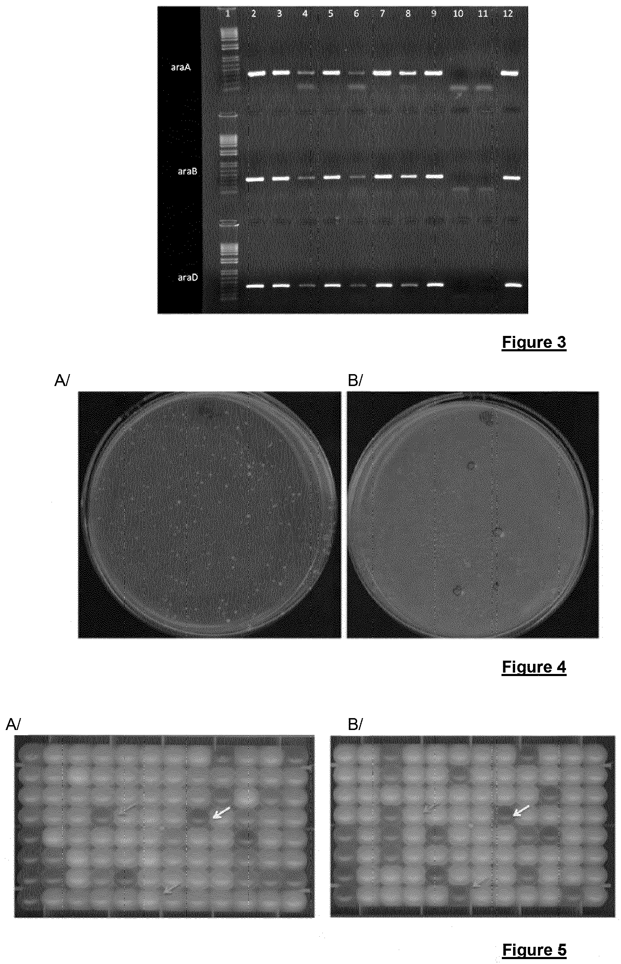

Les résultats, présentés à la figure 3, montrent qu'un produit de PCR a effectivement été amplifié pour les 3 gènes de la voie arabinose dans tous les transformants testés (8). Ce constat suggère que ces gènes ont effectivement été intégrés dans le génome des levures.

Par ailleurs, un séquençage classique a été réalisé afin de vérifier que les gènes *araA, B* et *D* ne sont pas mutés (données non présentées).

### 3. Sélection des souches réellement aptes à métaboliser l'arabinose :

Afin de poursuivre plus avant les investigations, il a été choisi de simplifier le système d'expression de la voie arabinose. Pour cela, la cassette a été modifiée pour exprimer les gènes *ara A, B* et *D* tout en étant dépourvue de séquence codant pour un transporteur à l'arabinose (*araT*)*.*

Afin de mesurer la fréquence d'apparition de souches aptes à métaboliser l'arabinose, la souche I-4953, déposée à la CNCM le 29 janvier 2015, a été transformée à l'aide de 1,3 µg de cette cassette portant *araA, B et D.*

Le produit de transformation a été étalé sur 2 types de milieux :
- un premier milieu correspondant à du YPG (10 g/L d'extrait de levure ; 10 g/L de peptone ; 20 g/L de glucose comme seule source de carbone) contenant de la Blasticidine à hauteur de 50 mg/L. Le but est de déterminer le nombre de cellules qui ont intégré la cassette dans leur génome ;
- un second milieu appelé YNB-Ara correspondant à un milieu minimal (YNB Difco^{®}) qui contient 10 g/L d'arabinose comme seule source de carbone. Ce milieu doit permettre de déterminer le nombre de cellules qui ont acquis le phénotype [ara+].

Le milieu YNB Difco^{®} (« Yeast Nitrogen Base without amino acids and ammonium sulfate »), référencé sous le numéro 0335-15, contient :
- Biotine 2 µg/L
- Pantothénate (calcium) 400 µg/L
- Acide folique 2 µg/L
- Inositol 2000 µg/L
- Niacine 400 µg/L
- Acide p-aminobenzoïque 200 µg/L
- Pyridoxine (chlorhydrate) 400 µg/L
- Riboflavine 200 µg/L
- Thiamine (chlorhydrate) 400 µg/L
- Acide borique 500 µg/L
- Sulfate de cuivre 40 µg/L
- Iodure de potassium 100 µg/L
- Chlorure de fer 200 µg/L
- Sulfate de manganèse 400 µg/L
- Molybdate de sodium 200 µg/L
- Sulfate de zinc 400 µg/L
- Phosphate de potassium monobasique 1 g/L
- Sulfate de magnésium 500 mg/L
- Chlorure de sodium 100 mg/L
- Chlorure de calcium 100 mg/L

pH final = 5.4
Sulfate d'ammonium (5 g/L)
La figure 4 illustre le résultat obtenu sur ces deux types de milieux.

Après 6 jours de croissance sur les deux milieux, les colonies ont été dénombrées. Il a été observé que le nombre de colonies sur milieu YNB-Ara représente environ 18 % de celui observé sur YPG + Blasticidine. En d'autres termes, seules 18 % des colonies ayant intégré les gènes *araA, B* et *D* dans leur génome semblent capables de pousser en utilisant l'arabinose comme seule source de carbone.

Il ressort de ce qui précède que :
- la cassette codant les gènes *araA, B* et *D* est nécessaire mais non suffisante pour obtenir un phénotype [ara+] ;
- la sélection directe des transformants sur un milieu contenant de l'arabinose comme seule source de carbone est possible et contrairement à l'enseignement de Wisselink et al. (2007; Appl Environ Microbiol 73, 4881-4891), une étape préalable de croissance en présence de galactose n'est pas nécessaire.

Pour la suite, la cassette a encore été simplifiée et dépourvue de tout marqueur de sélection, notamment de résistance aux antibiotiques.

1156 transformants ayant poussé sur le milieu YNB-Ara ont ensuite été testés en format Deep Well (= microplaque à 96 puits) afin de confirmer leur capacité à croitre en anaérobie sur arabinose, mais également pour vérifier le maintien de leur capacité à fermenter le xylose et le glucose.

La composition des milieux mis en œuvre dans ce cadre est détaillée ci-dessous :
Milieu YF =
- extrait de levure (EXL) 5 g/L ;
- di-amonium phosphate 4,7 g/L ;
- acide citrique 11,4 g/L ;
- citrate tri-sodique 13,5 g/L ;
- ZnSO₄ 21,2 mg/L ;
- MgSO₄ 7H₂O 1 g/L ;
- thiamine 18,24 mg/L ;
- pyridoxine 5,28 mg/L ;
- biotine 1,76 g/L ;
- panthoténate 3,8 mg/L ;
- acide nitotinique 20 mg/L ;
- mésoinositol 50 mg/L ;
- riboflavine 1 mg/L ;
- para-amino-benzoate 1,2 mg/L ;
- tween 80 1 g/L.

### Milieu YF-ara: milieu YF contenant 70 g/L d'arabinose

### Milieu YF-xylose: milieu YF contenant 70 g/L de xylose

Le pH du milieu est maintenu à 5. La culture est réalisée à 32 °C.

La figure 5 illustre les résultats obtenus avec une série de souches, dont la croissance a été suivie en parallèle sur un milieu contenant de l'arabinose (A) ou du xylose (B) comme seule source de carbone, ce dernier milieu permettant de sélectionner les souches ayant conservé leur capacité à métaboliser le xylose.

Les résultats présentés sur la figure 5 montrent que le mode de sélection des souches [ara+] a encore été amélioré : la figure 5A montre que 15 clones, parmi les 91 testés, ne semblent pas capables de recommencer à se multiplier en utilisant l'arabinose. Cela suggère que, contrairement à la sélection sur la base d'une résistance à un antibiotique, la sélection sur YNB-Ara permet d'obtenir 85 % de transformants qui ont acquis le phénotype [ara+].

Un autre volet issu des résultats présentés sur la figure 5 concerne la cohabitation des deux voies métaboliques xylose et arabinose. Ainsi, il est visible que certaines souches ont acquis le phénotype [ara+] au détriment du phénotype [xyl+]. D'autres, en revanche, n'ont pas conservé le phénotype [ara+] après les repiquages sur glucose (résultats non montrés). Toutefois, parmi les 91 souches testées, 67 semblent avoir acquis et conservé le double phénotype [ara+ ; xyl+], soit près des 3/4 des transformants.

### 4. Comparaison entre 2 souches sélectionnées :

### - Fermentation sur milieu YFCF :

Les performances de 2 clones isolés (appelées clones 13 et 126, respectivement), comme décrit ci-dessus, ont été comparées lors de la fermentation en milieu YFCF à pH 5 comprenant comme source de carbone du glucose, mais également de l'arabinose et du xylose équi-représentés, ainsi que de l'acide acétique, se rapprochant ainsi des milieux de fermentation naturels.

Plus précisément, la composition du milieu YFCF est la suivante :
- 10 g/L d'extrait de levure ;
- 10 g/L de bacto-peptone ;
- 63 g/L de glucose;
- 28 g/L de xylose;
- 28 g/L d'arabinose;
- 4 g/L d'acide acétique (quantité introduite dans le milieu de culture à pH 5).

Les conditions de fermentation sont les suivantes:
- pH : 5
- Température : 32°C
- Conditions en O₂ : sans apport d'oxygène
- Agitation : 100 rpm
- Durée de la culture : jusqu'à 72 heures
- Préculture / Incubation : 0,25 g/kg eq MS des levures préalablement propagées à saturation sur milieu YPG pendant 24 heures.

La production d'éthanol est estimée de manière indirecte par une mesure de la perte de masse de la fiole de fermentation, cette perte de masse étant directement corrélée à la production de CO₂ qui est stœchiométrique avec celle d'alcool. Elle est exprimée en g par kg de milieu.

Les concentrations en glucose, xylose, arabinose et glycérol dans le milieu sont suivies par HPLC.

Les variations de biomasse sont évaluées par la mesure de la matière sèche résiduelle après 4 heures à 105°C.

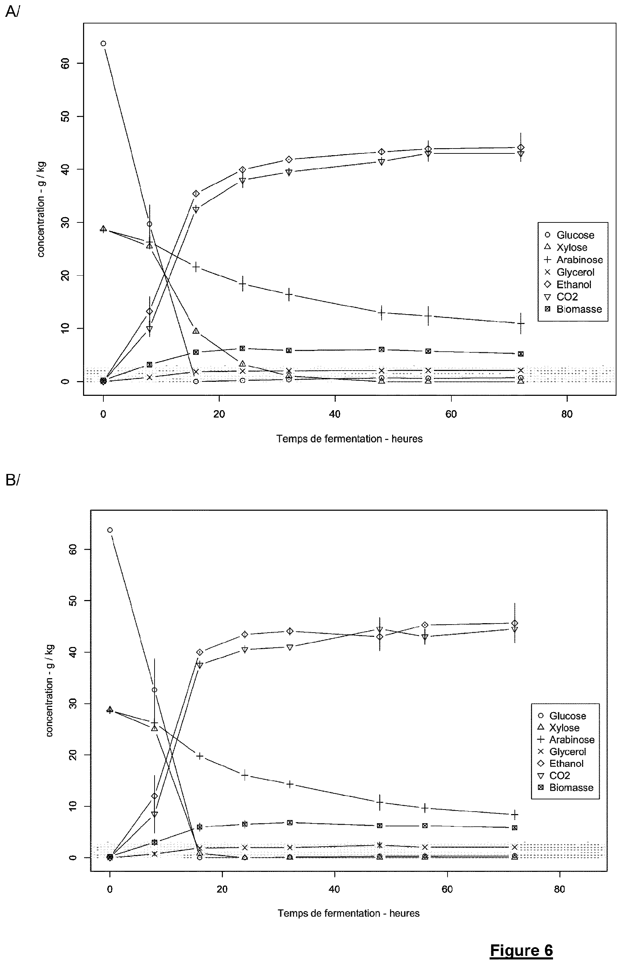

Les résultats, présentés à la figure 6, révèlent :
- une réduction de la vitesse de consommation du xylose pour le clone 13 par rapport au clone 126, assorti également d'une moindre vitesse de production d'éthanol ;
- de manière notable, une vitesse de consommation de l'arabinose moins importante dans le clone 13 que dans le clone 126. Ainsi, après 32 heures de fermentation, le clone 13 a consommé 12,1 +/- 0,6 g/kg d'arabinose, alors que le clone 126 en a consommé 15,3 +/- 0,5 g/kg.

Il est également à noter que les pentoses sont consommés alors que la concentration en glucose extracellulaire n'est pas nulle.

Il a ensuite été tenté d'expliquer la différence observée entre les clones 13 et 126, en particulier au niveau de la vitesse de fermentation de l'arabinose.

### Analyse des activités de la voie :

L'activité d'enzymes intracellulaires impliquées dans les voies métaboliques de l'arabinose et du xylose ont été testées.

Tout d'abord, aucune différence notable n'a été notée au niveau de l'activité xylitol déshydrogénase (résultats non montrés).

En revanche, il a été observé un profil de l'activité arabinose isomérase (codée par *araA)* différent entre les clones 13 et 126, avec une inhibition compétitive plus importante pour le clone 13 (résultats non montrés).

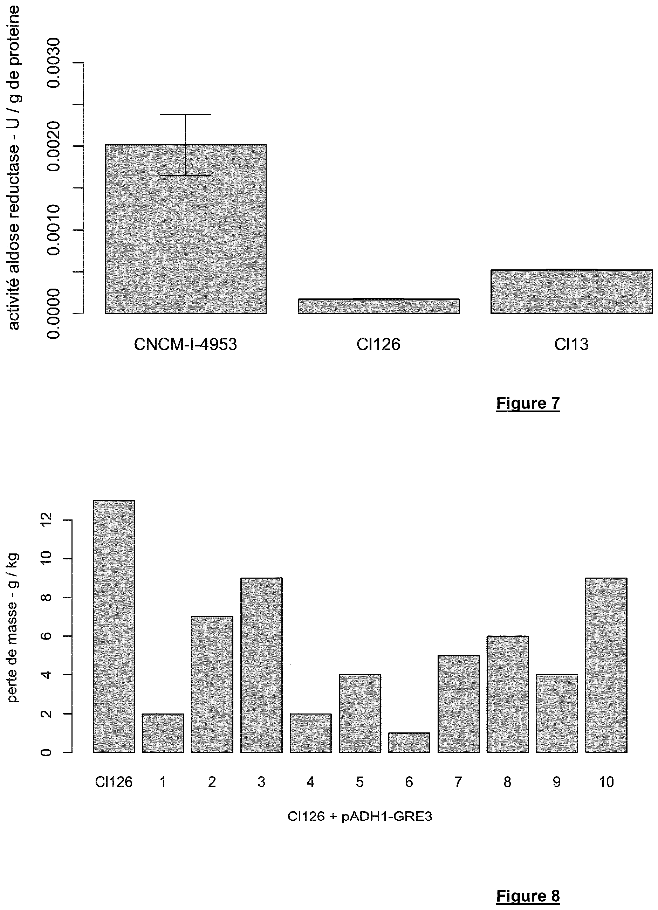

Comme illustrée à la figure 7, cette différence a pu être corrélée à une différence au niveau de la réduction des aldoses, et plus précisément de l'activité aldose réductase de ces deux clones. A noter que dans la souche de départ I-4953, le gène *GRE3* codant l'aldose réductase principale chez *S. cerevisiae* a été délété mais qu'il reste une activité résiduelle (voir Figure 7).

### Dosage de l'activité aldose réductase dans un extrait cellulaire :

### Propagation des souches

Les souches à tester sont mises à croître en milieu riche (YPG). Deux phases de propagation successives de 24h à 30°C, sous agitation, sont nécessaires pour obtenir suffisamment de biomasse. Une fois les récoltes des biomasses effectuées, la mesure de la matière sèche est réalisée sur 1 ml de chaque crème après passage à l'étuve à 105°C pendant 4 heures. Les fermentations sont réalisées dans des ballons de 250 ml contenant 100 g de milieu de fermentation YF-xylose. Chaque ballon est ensemencé à raison de 0.5g/kg équivalent matière sèche. Les fermentations se déroulent à 32°C, sous agitation à 110 rpm et durent 3 jours. A ce stade, 50 ml de produit de fermentation sont collectés et servent ensuite pour la détermination des différentes activités enzymatiques et de la concentration protéique de l'échantillon.

### Préparation de l'extrait cellulaire

50 ml de produit de fermentation sont collectés par centrifugation (4700 rpm, 2 min, 4°C) puis resuspendus dans 3 ml de tampon phosphate de potassium 0.1M, pH 7. Après une autre centrifugation, chaque culot est ensuite resuspendu dans 1ml de tampon phosphate de potassium 0.1M, pH 7.

1 ml de culot resuspendu est ensuite transféré dans 1 tube pour Fast Prep (préalablement rempli avec 250 µl de billes) puis broyé à 4°C : 4 x 30 secondes (6 m/s), espacés de 30 secondes chacun. Ensuite, le broyat est centrifugé 3 minutes à 10000 rpm (pour faire tomber les billes ainsi que les débris cellulaires) et la totalité du surnageant est transférée dans un tube Eppendorf refroidi sur glace.

### Dosage des activités enzymatiques

Le dosage de l'activité enzymatique se fait par un suivi de l'absorbance à 340 nm. Les mesures sont réalisées dans un environnement thermostaté à 32°C.

| | blanc | échantillon (µl) |
|---|---|---|
| Tampon phosphate (KH2PO4/K2HPO4 0.1M pH=7) | 750 | 750 |
| NAD (0,04M) | | 100 |
| extrait cellulaire | | 20 |
| eau | 750 | 430 (qsp 1300) |
| xylitol 2M | | 200 |

Le substrat est ajouté après 1 minute.

Les résultats présentés à la figure 7 mettent en évidence une très forte baisse de l'activité aldose réductase dans les clones 13 et 126 par rapport à la souche parentale I-4953. Il est cependant à noter que cette activité est deux fois plus importante dans le clone 13 que dans le clone 126. Cette observation semble pouvoir expliquer les piètres performances du clone 13 par rapport au clone 126. Afin de pouvoir corroborer ce résultat avec les performances en fermentation du xylose et/ou de l'arabinose, il a été tenté d'augmenter l'activité aldose réductase dans le clone 126.

### Impact de la réintroduction d'un gène codant une aldose réductase sur la capacité des cellules à métaboliser l'arabinose :

Il ressort que deux des principales différences entre les clones 126 et 13 résident dans leur capacité à fermenter le xylose et l'arabinose et dans leur capacité à les réduire en xylitol et en arabitol, respectivement. Dans la mesure où ces deux phénotypes semblent anti-corrélés, il a été tenté de renforcer cette activité dans les cellules du clone 126.

En ce sens, le gène *GRE3* qui code pour la principale aldose réductase chez la levure *S. cerevisiae* (Garay-Arroyo and Covarrubias, 1999, Yeast 15, 879-892; Träff et al., 2001 Appl. Environ. Microbiol. 67, 5668-5674) a été réintroduit. En pratique, une copie du gène *GRE3* placé sous la dépendance du promoteur du gène *ADH1* a été intégrée au niveau du locus natif de *GRE3* dans le génome du clone 126.

Afin de valider la bonne intégration du gène au bon locus, les amorces suivantes ont été utilisées dans des réactions de PCR :
- B6C2 (CCTATTGCTGTTTCCTCTTCAAAGTAC ; SEQ ID NO: 7), s'hybridant dans le terminateur du marqueur de sélection ;
- B13B1 (TAGTTGTCAGTGCAATCCTTC ; SEQ ID NO: 8), complémentaire d'une région du terminateur du gène *GRE3.*

Après vérification de l'intégration de la cassette dans le génome des transformants sélectionnés (résultats non montrés), leur capacité à utiliser l'arabinose comme seule source de carbone en anaérobie a été testée. En ce sens, les transformants ainsi que le clone 126 ont été inoculés à hauteur de 2 g (eq MS) / kg dans du milieu YF-Ara.

La perte de masse après 48 heures de fermentation est représentée à la figure 8. Ces résultats montrent que tous les clones ayant intégré le gène *GRE3* ont une capacité à fermenter l'arabinose qui est amoindrie par rapport à celle du clone 126. Ce résultat conforte donc l'hypothèse selon laquelle une trop forte activité aldose réductase limite la capacité des cellules à fermenter l'arabinose.

### CONCLUSIONS :

L'intégration de la voie de fermentation de l'arabinose, en l'occurrence des gènes *araA (B. licheniformis)* / *araB (E. coli)* / *araD (E. coli)* au niveau du locus *HO* de la souche I-4953 de *S. cerevisiae,* suivi d'un criblage sur un milieu contenant de l'arabinose comme seule source de carbone, a permis de sélectionner des souches aptes à fermenter l'arabinose. En outre, il a été montré que parmi ces souches, les plus favorables étaient celles qui présentaient une activité aldose réductase faible. Dans ce cadre, il a été isolé une souche particulièrement intéressante, à savoir la souche EG31 qui a été déposée auprès de l'Institut Pasteur (Collection Nationale de Cultures de Microorganismes, 25 rue du Docteur Roux, 75724 Paris Cedex 15) sous le numéro CNCM I-5085 en date du 19 Mai 2016.

### II/ Obtention d'une souche de Saccharomyces cerevisiae optimisée pour la fermentation de l'arabinose et du xvlose :

### 1. Sélection d'une souche capable de fermenter l'arabinose et le xvlose par évolution dirigée :

Dans le but de sélectionner une souche encore optimisée pour la fermentation des pentoses, et en particulier de l'arabinose, la souche EG31 a été soumise à une évolution dirigée en batch.

Ainsi, il a été mis au point un protocole d'évolution dirigée permettant d'obtenir le phénotype recherché. Pour cela, il a été défini et mis en œuvre un milieu dans lequel les levures qui ne consommeraient qu'un seul des deux sucres seraient désavantagées. En pratique, dans ce premier milieu nommé « GO xylose arabinose » et défini ci-dessous, ce n'est pas la source d'azote qui est limitante mais ce sont les sources de carbone. En pratique, ce milieu contient 10 g/L de (NH₄)₂PO₄ mais seulement 4 g/L de xylose et 5 g/L d'arabinose.

### Milieu 1 : GO xylose arabinose (pH 5):

- Xylose 4 g/L
- Arabinose 5 g/L
- (NH4)2HPO4 (DAP) 10 g/L
- Acide citrique 11;4 g/L
- Citrate trisodique 13,5 g/L
- ZnSO4 0,004 g/L
- MgSO4 0,5 g/L
- KH2PO4 1 g/L
- NaCl 0,1 g/L
- CaCl2 0,1 g/L
- CuSO4 0,00006 g/L
- H3BO3 0,0005 g/L
- KI 0,0001 g/L
- MnSO4 0,0004 g/L
- Na2MoO4 0,0002 g/L
- FeCl3 0,0002 g/L
- Pyridoxine 0,002 g/L
- Biotine 0,0008 g/L
- Panthoténate 0,002 g/L
- Acide nicotinique 0,001 g/L
- Mésoinositol 0,001 g/L
- Tween 80 1 g/L

La culture est réalisée à 32°C sous une agitation de 100 rpm dans des fioles obturées par des bouchons qui permettent de réduire l'apport d'oxygène dans le milieu et de laisser échapper le CO₂ en surpression qui est produit tout au long de cette culture. La culture dure environ 7 jours dans ces conditions.

### 2. Sélection d'une souche avant en outre conservée sa capacité à fermenter le glucose et/ou le xvlose en présence d'acide acétique

Afin d'anticiper un éventuel risque de perte de tolérance vis-à-vis des inhibiteurs, en particulier de l'acide acétique sous forme non dissociée, il a été introduit, dans le cycle d'évolution dirigée, deux cultures successives en batch dans des milieux contenant soit du glucose, soit du xylose, comme seule source de carbone et en présence, dans les deux cas, de fortes concentrations en acide acétique.

### Milieu 2 : YF-glucose acide :

Le milieu YF-glucose acide correspond au milieu YF décrit ci-dessus, contenant 150 g/L de glucose et présentant une concentration en acide acétique (quantité introduite dans le milieu de culture à pH 4,4) égale à 5 g/L.

### Milieu 3 : YF-xylose acide :

Le milieu YF-xylose acide correspond au milieu YF-xylose décrit ci-dessus, avec une concentration en acide acétique (quantité introduite dans le milieu de culture à pH 5) égale à 4 g/L.

### Conditions de culture :

La culture est réalisée à 32 °C sous une agitation de 100 rpm dans des fioles obturées par des bouchons qui permettent de réduire l'apport d'oxygène dans le milieu et de laisser échapper le CO₂ en surpression qui est produit tout au long de cette culture. La culture dure environ 7 jours dans ces conditions.

### 3. Elimination des « petites » :

Afin d'éviter la sélection de souches dépourvues de mitochondries, qui ne seraient pas compatible avec les processus de production industriels, il a également été ajouté une étape de culture sur un milieu contenant du glycérol comme seule source de carbone. Cette étape nécessite la présence d'une chaine de transfert d'électron fonctionnelle dans les cellules pour qu'elles puissent se multiplier.

### Milieu 4 : YNB glycérol :

- 3,4 g / L de « yeast nitrogen base » DIFCO^{®} ;
- 5 g / L sulfate d'ammonium ;
- 10 g/ L de glycérol comme seule source de carbone.

La culture est réalisée à 30°C sous une agitation à 150 rpm dans des fioles à baffles obturées par des bouchons poreux qui permettent l'apport d'oxygène dans le milieu. La culture dure environ 24 à 48 heures dans ces conditions.

En pratique, un cycle d'évolution dirigée se caractérise donc par :
- une culture sur milieu GO xylose arabinose ; puis
- une culture sur milieu YF glucose acide ; puis
- une culture sur milieu YF xylose acide ; puis
- une culture sur milieu YNB glycérol.

### 4. Evaluation des clones obtenus :

En pratique, deux cycles de culture ont été réalisés.

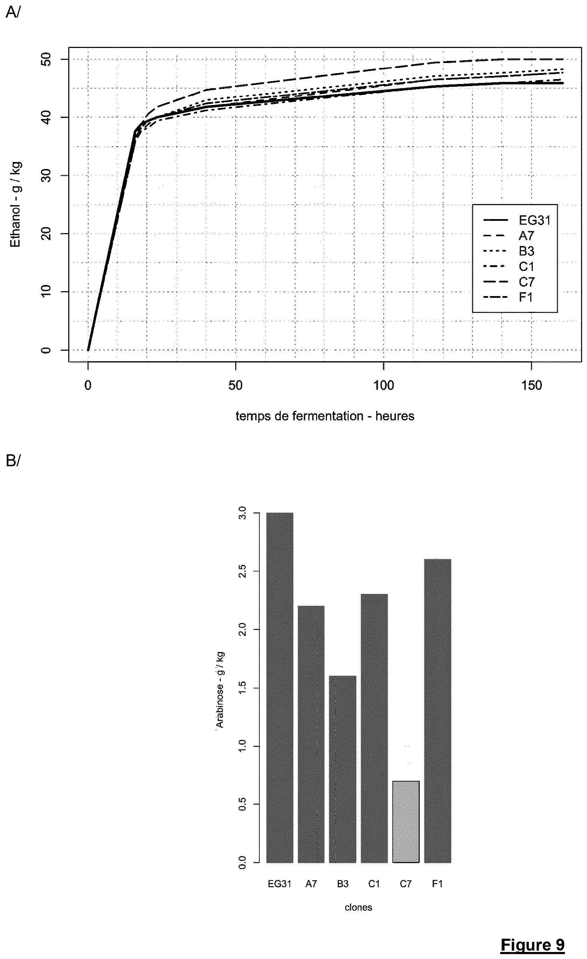

A l'issue de cette évolution dirigée, les clones ont été individualisés sur des boites de milieu complet et les performances des clones obtenus ont été évaluées sur un milieu YFCF dont la composition est donnée ci-dessus. La figure 9A montre l'évolution de la production d'éthanol en fonction du temps de fermentation pour 6 des meilleurs clones obtenus.

Ces résultats montrent que 5 des 6 clones ont une cinétique proche de celle de la souche EG31, avec toutefois un titre alcoolique final supérieur à celui de la souche EG31. Le dernier clone noté C7 semble, quant à lui, plus rapide lors de la transition entre le métabolisme des sucres à 6 carbones vers celui des sucres à 5 carbones. Par la suite, la cinétique de fermentation semble la même que celle des autres clones. A la fin de la fermentation, il semble que le titre alcoolique soit plus important avec cette souche que lors de la mise en œuvre des autres clones.

Afin de valider que cette amélioration tient effectivement à une meilleure consommation de l'arabinose, un dosage par HPLC a été réalisé afin de quantifier les sucres résiduels. La figure 9B représente les concentrations en arabinose dans le milieu de fermentation après 160 heures de fermentation. Les résultats confirment que tous les clones sélectionnés à l'issue de l'évolution dirigée ont consommé davantage d'arabinose que la souche EG31, le clone noté C7 apparaissant comme le plus performant. Ce clone a été rebaptisé souche EG32 qui a été déposée auprès de l'Institut Pasteur (Collection Nationale de Cultures de Microorganismes, 25 rue du Docteur Roux, 75724 Paris Cedex 15) sous le numéro CNCM I-5086 en date du 19 Mai 2016.

## Revendications

1. Procédé d'obtention et de sélection de souches de levure capables de fermenter l'arabinose, le glucose et le xylose en présence d'acide acétique sous forme non dissociée comprenant :
- l'intégration chromosomique des gènes *araA, araB* et *araD,* encore plus avantageusement des gènes *araA* de *B. licheniformis, araB* et *araD d'E. coli,* dans une souche de levure capable de fermenter le xylose en présence d'acide acétique sous forme non dissociée et présentant au moins un gène *GRE3* inactivé ou délété ;
- la mise en culture directe des souches transformées dans un milieu contenant de l'arabinose comme seule source de carbone pour la sélection des souches aptes à métaboliser ledit arabinose ;
- optionnellement la sélection des souches transformées et ayant métabolisé l'arabinose pour leur activité aldose réductase inférieure à 0,002 U / g de protéine, voire inférieure ou égale à 0,0005 U / g de protéine.

2. Procédé selon la revendication 1 ***caractérisé* en ce que** la souche de levure capable de fermenter le xylose en présence d'acide acétique sous forme non dissociée et présentant au moins un gène *GRE3* inactivé ou délété est choisie parmi *Saccharomyces, Schizosaccharomyces, Pichia, Yarrowia, Paffia, Kluyveromyces, Candida, Talaromyces, Brettanomyces, Pachysolen, Hansenula, Kloeckera, Schwanniomyces* et *Debaryomyces,* avantageusement une souche de *Saccharomyces cerevisiae.*

3. Procédé selon la revendication 2 ***caractérisé* en ce que** la souche de levure capable de fermenter le xylose en présence d'acide acétique sous forme non dissociée et présentant au moins un gène *GRE3* inactivé ou délété est la souche déposée à la CNCM en date du 29 Janvier 2015 sous le numéro I-4953.

4. Souche de levure obtenue à l'aide du procédé selon l'une des revendications 1 à 3 ***caractérisée* en ce qu'**elle présente en outre une activité aldose réductase inférieure à 0,002 U / g de protéine, voire inférieure ou égale à 0,0005 U / g de protéine, et qu'il s'agit de la souche I-5085 déposée auprès de la CNCM (Collection Nationale de Cultures de Microorganismes, Institut Pasteur, 25 rue du Docteur Roux, 75724 Paris Cedex 15) en date du 19 Mai 2016.

5. Procédé d'obtention et de sélection d'une souche de levure présentant une capacité améliorée à fermenter l'arabinose, le glucose et le xylose en présence d'acide acétique sous forme non dissociée, dans lequel une souche de levure présentant une capacité à fermenter l'arabinose, le glucose et le xylose en présence d'acide acétique sous forme non dissociée et obtenue à l'aide du procédé selon l'une des revendications 1 à 3, avantageusement une souche selon la revendication 4, est cultivée successivement dans les conditions suivantes :
- une culture en anaérobiose ou en hypoxie dans un milieu contenant, comme seules sources de carbone, de l'arabinose et du xylose en concentrations limitantes pour la production de biomasse, avantageusement à hauteur de 5 g/L et 4 g/L, respectivement ; puis
- deux cultures successives en anaérobiose ou en hypoxie et en présence d'acide acétique sous forme non dissociée, l'une dans un milieu contenant du glucose comme seule source de carbone et l'autre dans un milieu contenant du xylose comme seule source de carbone ;
- éventuellement une culture en aérobiose dans un milieu minimum contenant comme seule source de carbone une source de carbone respiratoire stricte, avantageusement du glycérol.

6. Procédé selon la revendication 5 ***caractérisé* en ce que** la culture successive de la souche dans les différents milieux est répétée au moins 2 fois.

7. Souche de levure susceptible d'être obtenue à l'aide du procédé selon l'une des revendications 5 à 6 ***caractérisée* en ce qu'**il s'agit de la souche I-5086, déposée auprès de la CNCM (Collection Nationale de Cultures de Microorganismes, Institut Pasteur, 25 rue du Docteur Roux, 75724 Paris Cedex 15) en date du 19 Mai 2016.

8. Souche de levure selon la revendication 4 ou 7 ***caractérisée* en ce qu'**elle possède au moins une copie surnuméraire du gène *HAA1,* avantageusement insérée au niveau du gène *GRE3.*

9. Utilisation d'une souche selon l'une des revendications 4, 7 et 8, pour la fermentation d'un matériau, contenant avantageusement de l'arabinose et/ou du xylose, et/ou pour la production d'éthanol.

10. Procédé de production de produits de fermentation ou d'éthanol comprenant les étapes suivantes :
- Incubation d'un matériau ou milieu contenant de l'arabinose et/ou du xylose avec une souche selon l'une des revendications 4, 7 et 8 ;
- Fermentation en condition anaérobie ou semi-anaérobie ;
- Récupération du ou des produits de fermentation, ou de l'éthanol.

11. Procédé de production de produits de fermentation ou d'éthanol selon la revendication 10, ***caractérisé* en ce que** le matériau ou milieu contient également du glucose.

12. Procédé de production de produits de fermentation ou d'éthanol selon la revendication 10 ou 11, ***caractérisé* en ce que** le matériau ou milieu est de l'hémicellulose ou des « corn fibers ».

## Patentansprüche

1. Verfahren zur Erzeugung und Auswahl von Hefestämmen, die in der Lage sind Arabinose, Glucose und Xylose in Anwesenheit von Essigsäure in nicht dissoziierter Form zu fermentieren, das umfasst:
- die Chromosomintegration der Gene *araA, araB* und *araD,* noch besser der Gene *araA* von *B. licheniformis, araB* und *araD* von E. *coli,* in einen Hefestamm, der Xylose in Anwesenheit von Essigsäure in nicht dissoziierter Form fermentieren kann und mindestens ein inaktiviertes oder deletiertes *GRE3* Gen aufweist;
- die direkte Anzucht der transformierten Stämme in einem Milieu mit Arabinose als einziger Kohlenstoffquelle zur Selektion von Stämmen, die diese Arabinose metabolisieren können;
- optional die Selektion der transformierten Stämme, die die Arabinose metabolisiert haben, aufgrund ihrer Aldose- Reduktase - Aktivität von kleiner als 0,002 U / g Protein, oder sogar kleiner oder gleich 0,0005 U / g Protein.

2. Verfahren nach Anspruch 1, **dadurch gekennzeichnet, dass** der Hefestamm, der Xylose in Anwesenheit von Essigsäure in nicht dissoziierter Form fermentieren kann und mindestens ein inaktiviertes oder deletiertes *GRE3* Gen aufweist, ausgewählt wird aus *Saccharomyces, Schizosaccharomyces, Pichia, Yarrowia, Paffia, Kluyveromyces, Candida, Talaromyces, Brettanomyces, Pachysolen, Hansenula, Kloeckera, Schwanniomyces* und *Debaryomyces,* vorteilhafterweise ein Stamm von *Saccharomyces cerevisiae.*

3. Verfahren nach Anspruch 2 **dadurch gekennzeichnet, dass** es sich bei dem Hefestamm, der Xylose in Anwesenheit von Essigsäure in nicht dissoziierter Form metabolisieren kann und mindestens ein inaktiviertes oder deletiertes *GRE3* Gen aufweist, um den Stamm handelt, der bei der CNCM mit Datum vom 29. Januar 2015 unter der Nummer 1-4953 registriert wurde.

4. Hefestamm erzeugt mit Hilfe des Verfahrens nach einem der Ansprüche 1 bis 3 **dadurch gekennzeichnet, dass** er außerdem eine Aldose- Reduktase - Aktivität von kleiner als 0,002 U / g Protein oder sogar kleiner oder gleich 0,0005 U / g Protein aufweist, und dass es sich um den Stamm 1-5085 handelt, registriert bei der CNCM (Collection Nationale de Cultures de Microorganismes, Institut Pasteur, 25 rue du Docteur Roux, 75724 Paris Cedex 15) mit Datum vom 19. Mai 2016.

5. Verfahren zur Erzeugung und Auswahl eines Hefestamms, der eine verbesserte Fähigkeit zur Fermentierung von Arabinose, Glucose und Xylose in Anwesenheit von Essigsäure in nicht dissoziierter Form aufweist, bei dem ein Hefestamm mit, der Fähigkeit zur Fermentierung von Arabinose, Glucose und Xylose in Anwesenheit von Essigsäure in nicht dissoziierter Form und erzeugt mit Hilfe des Verfahrens nach einem der Ansprüche 1 bis 3, vorteilhafterweise ein Stamm nach Anspruch 4, nacheinander unter den folgenden Bedingungen kultiviert wird:
- Kultivierung unter anaeroben Bedingungen oder unter Hypoxie in einem Milieu, das als einzige Kohlenstoffquellen Arabinose und Xylose enthält, in Konzentrationen, die für die Erzeugung von Biomasse einschränkend wirken, vorteilhafterweise in Höhe von jeweils 5 g/L und 4 g/L; dann
- zwei aufeinanderfolgende Kultivierungen unter anaeroben Bedingungen oder unter Hypoxie in Anwesenheit von Essigsäure in nicht dissoziierter Form, die eine in einem Milieu, das als einzige Kohlenstoffquellen Glucose enthält, die andere in einem Milieu, das als einzige Kohlenstoffquellen Xylose enthält;
- eventuell eine Kultivierung unter aeroben Bedingungen in einem MinimalMedium, das als einzige Kohlenstoffquellen eine strikt respiratorische Kohlenstoffquelle enthält, vorteilhafterweise Glyzerin.

6. Verfahren nach Anspruch 5, **dadurch gekennzeichnet, dass** die aufeinanderfolgende Kultivierung des Stamms in den verschiedenen Milieus mindestens 2 Mal wiederholt wird.

7. Hefestamm erzeugt mit Hilfe des Verfahrens nach einem der Ansprüche 5 bis 6 **dadurch gekennzeichnet, dass** es sich um den Stamm 1-5086 handelt, registriert bei der CNCM (Collection Nationale de Cultures de Microorganismes, Institut Pasteur, 25 rue du Docteur Roux, 75724 Paris Cedex 15) mit Datum vom 19. Mai 2016.

8. Hefestamm nach Anspruch 4 oder 7 **dadurch gekennzeichnet, dass** er mindestens eine überzählige Kopie des *HAAI* Gens enthält, vorteilhafterweise eingefügt in Höhe des *GRE3* Gens.

9. Einsatz eines Stamms nach einem der Ansprüche 4, 7 und 8, zur Fermentierung eines Materials, das vorteilhafterweise Arabinose und/ oder Xylose enthält und/ oder zur Erzeugung von Ethanol.

10. Verfahren zur Erzeugung von Fermentierungsprodukten oder Ethanol, das die folgenden Schritte enthält:
- Inkubation eines Materials oder Milieus, das Arabinose und/oder Xylose enthält, mit einem Stamm nach einem der Ansprüche 4, 7 und 8;
- Fermentierung unter anaeroben oder halb-anaeroben Bedingungen;
- Auffangen des oder der Fermentierungsprodukte oder des Ethanols.

11. Verfahren zur Erzeugung von Fermentierungsprodukten oder Ethanol nach Anspruch 10, **dadurch gekennzeichnet, dass** das Material oder Milieu auch Glucose enthält.

12. Verfahren zur Erzeugung von Fermentierungsprodukten oder Ethanol nach Anspruch 10 oder 11, **dadurch gekennzeichnet, dass** es sich bei dem Material oder Milieu um Hemicellulose oder « corn fibers » handelt.

## Claims

1. A process for obtaining and selecting yeast strains able to ferment arabinose, glucose and xylose in the presence of acetic acid in non-dissociated form comprising:
- the chromosomal integration of *araA, araB* and *araD* genes, advantageously *araA* of *B. licheniformis, araB* and *araD* of *E. Coli,* in a yeast strain able to ferment xylose in the presence of acetic acid in non-dissociated form and having at least one inactivated or deleted *GRE3* gene;
- putting the transformed strains into a direct culture in a medium containing arabinose as the sole carbon source for the selection of strains able to metabolize the said arabinose;
- optionally selecting transformed strains having metabolized arabinose for their aldose reductase activity less than or equal to 0.002 U / g protein, or even less than or equal to 0.0005 U / g of protein.

2. The process according to claim 1, **characterized in that** the yeast strain able to ferment xylose in the presence of acetic acid in non-dissociated form and having at least one inactivated or deleted *GRE3* gene is chosen from among *Saccharomyces, Schizosaccharomyces, Pichia, Yarrowia, Paffia, Kluyveromyces, Candida, Talaromyces, Brettanomyces, Pachysolen, Hansenula, Kloeckera, Schwanniomyces* and *Debaryomyces,* advantageously a *Saccharomyces cerevisiae* strain.

3. The process according to claim 2, **characterized in that** the yeast strain able to ferment xylose in the presence of acetic acid in non-dissociated form and having at least one inactivated or deleted *GRE3* gene is the strain registered at the CNCM on January 29, 2015 under number 1-4953.

4. A yeast strain obtained using the process according to one of claims 1 to 3, **characterised in that** it further presents an aldose reductase activity less than or equal to 0.002 U / g of protein, or even less than or equal to 0.0005 U / g of protein and is the strain 1-5085, registered at the CNCM (Collection Nationale de Cultures de Microorganismes, Institut Pasteur, 25 rue du Docteur Roux, 75724 Paris Cedex 15) on May 19, 2016.

5. A process for obtaining and selecting a yeast strain with an improved ability to ferment arabinose, glucose and xylose in the presence of acetic acid in non-dissociated form, wherein a yeast strain able to ferment arabinose, glucose and xylose in the presence of acetic acid in non-dissociated form and obtained using the process according to one of claims 1 to 3, advantageously a strain according to claim 4, is successively cultivated under the following conditions:
- an anaerobic or hypoxia culture in a medium containing, as sole carbon sources, arabinose and xylose in limiting concentrations for the production of biomass, advantageously in an amount of 5 g/L and 4 g/L respectively; then
- two successive anaerobic or hypoxia cultures in the presence of acetic acid in non-dissociated form, one in a medium containing glucose as the sole carbon source and the other in a medium containing xylose as the sole carbon source;
- possibly an aerobic culture in a minimum medium containing, as the sole carbon source, a source of strict respiratory carbon, advantageously glycerol.

6. The process according to claim 5, **characterized in that** the successive culture of the strain in the various media is repeated at least 2 times.

7. A yeast strain able to be obtained with the process according to one of claims 5 to 6, **characterized in that** it is strain 1-5086, registered at the CNCM (Collection Nationale de Cultures de Microorganismes, Institut Pasteur, 25 rue du Docteur Roux, 75724 Paris Cedex 15) on May 19, 2016.

8. The yeast strain according to one of claim 4 or 7, **characterized in that** it has at least one supernumerary copy of the *HAA1* gene, advantageously inserted at the level of the *GRE3* gene.

9. Use of a strain according to one of claims 4, 7 and 8 for fermentation of a material, advantageously containing arabinose and/or xylose, and/or for the production of ethanol.

10. A process for the production of fermentation products or ethanol comprising the following steps:
- Incubation of a material or medium containing arabinose and/or xylose with a strain according to one of claims 4, 7 and 8;
- Fermentation under anaerobic or semi-anaerobic conditions;
- Recovery of one or more fermentation product(s), or ethanol.

11. The process for production of fermentation products or ethanol according to claim 12, **characterized in that** the material or medium also contains glucose.

12. The process for production of fermentation products or ethanol according to claim 10 or 11, **characterized in that** the material or medium is hemicellulose or "corn fibers".


### SEQUENCE LISTING

<110> LESAFFRE ET COMPAGNIE
<120> OBTENTION DE SOUCHES DE LEVURE PERFORMANTES POUR LA METABOLISATION DE
   L\222ARABINOSE
<130> L220-B-49632 PCT
<150> FR1750550
   <151> 2017-01-24
<160> 8
<170> BiSSAP 1.3.2
<210> 1
   <211> 19
   <212> DNA
   <213> Artificial Sequence
<223> "araA-v-f-1 "
<220>
   <223> araA-v-f-1
<400> 1
   atgattcaag ctaagaccc 19
<210> 2
   <211> 20
   <212> DNA
   <213> Artificial Sequence
<223> "araA-v-r-1 "
<220>
   <223> araA-v-r-1
<400> 2
   atgtcaatct tgtcccatgg 20
<210> 3
   <211> 20
   <212> DNA
   <213> Artificial Sequence
<223> "araB-v-f-1 "
<220>
   <223> araB-v-f-1
<400> 3
   atggctattg ctattggttt 20
<210> 4
   <211> 20
   <212> DNA
   <213> Artificial Sequence
<223> "araB-v-r-2 "
<220>
   <223> araB-v-r-2
<400> 4
   ccacaaaacg aacatagcgt 20
<210> 5
   <211> 20
   <212> DNA
   <213> Artificial Sequence
<223> "araD-v-f-1 "
<220>
   <223> araD-v-f-1
<400> 5
   atgttggaag acttgaagag 20
<210> 6
   <211> 20
   <212> DNA
   <213> Artificial Sequence
<223> "araD-v-r-1 "
<220>
   <223> araD-v-r-1
<400> 6
   tagaagtagt cagcgtgggt 20
<210> 7
   <211> 27
   <212> DNA
   <213> Artificial Sequence
<223> "B6C2"
<220>
   <223> B6C2
<400> 7
   cctattgctg tttcctcttc aaagtac 27
<210> 8
   <211> 21
   <212> DNA
   <213> Artificial Sequence
<223> "B13B1"
<220>
   <223> B13B1
<400> 8
   tagttgtcag tgcaatcctt c 21